(19) Europäisches Patentamt
European Patent Office
Office européen des brevets

(11) **EP 4 648 002 A1**

(12) **EUROPEAN PATENT APPLICATION**
published in accordance with Art. 153(4) EPC

(43) Date of publication:
**12.11.2025 Bulletin 2025/46**

(21) Application number: **24738583.4**

(22) Date of filing: **06.01.2024**

(51) International Patent Classification (IPC):
**G06T 3/4053** (2024.01)

(52) Cooperative Patent Classification (CPC):
**C12Q 1/6869; G01N 21/01; G01N 21/84;
G02B 21/00; G02B 21/06; G06T 1/20;
G06T 3/4053; G06T 7/62; G06V 10/141;
G06V 10/96; G06V 10/98**

(86) International application number:
**PCT/CN2024/070990**

(87) International publication number:
**WO 2024/146652 (11.07.2024 Gazette 2024/28)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **06.01.2023 CN 202310022193**

(71) Applicant: **GeneMind Biosciences Company
Limited
Shenzhen, Guangdong 518023 (CN)**

(72) Inventor: **BO, En
Shenzhen, Guangdong 518023 (CN)**

(74) Representative: **Schiweck Weinzierl Koch
Patentanwälte Partnerschaft mbB
Ganghoferstraße 68 B
80339 München (DE)**

(54) **IMAGE RECONSTRUCTION METHOD AND APPARATUS**

(57)    An image reconstruction method and apparatus. The method comprises: acquiring a plurality of original images of a sample under test in a same field of view; and performing image reconstruction according to the plurality of original images in respect of said field of view, so as to obtain a reconstructed image of said field of view. Image reconstruction is performed on the basis of a plurality of original images of a same field of view, so that the image resolution can be improved.

Acquiring a plurality of raw images of a sample of interest in the same field of view ~ 1000

Performing image reconstruction based on the plurality of raw images for the field of view to obtain a reconstructed image of the field of view ~ 2000

FIG. 20

## Description

## TECHNICAL FIELD

The present disclosure relates to the field of image processing, and in particular, to a method for image reconstruction and an apparatus.

## BACKGROUND

Currently, mainstream Massive Parallel Sequencing (MPS) technologies are primarily based on optical microscopic imaging systems. These optical microscopic imaging systems enable the acquisition of images of samples of interest, and based on the images, the detection of the samples of interest can be completed, such as obtaining the base sequence of the samples of interest. However, optical microscopic imaging systems are subject to the optical diffraction limit resolution, which restricts the resolution of the images acquired and consequently limits the improvement of sequencing throughput. Therefore, enhancing image resolution is an issue that warrants attention.

## SUMMARY

To address the above issue, the present disclosure provides a method for image reconstruction and a sequencing apparatus, which are specifically explained hereinafter.

According to a first aspect, an embodiment provides a method for image reconstruction. The method includes:

acquiring a plurality of raw images of a sample of interest in a same field of view; and

performing image reconstruction based on the plurality of raw images for the field of view to obtain a reconstructed image of the field of view.

According to the method for image reconstruction and the sequencing apparatus of the above embodiment, reconstructing an image based on a plurality of raw images in the same field of view can improve the resolution of the image.

According to another aspect, an apparatus is provided. The apparatus includes a memory and a processor. The memory is configured to store programs, and the processor is configured to implement the above method for image reconstruction by running the programs stored in the memory.

## BRIEF DESCRIPTION OF THE DRAWINGS

FIG. 1 is a schematic diagram of the microscopic structure of a light modulator according to an embodiment;

FIG. 2(a) is a schematic diagram illustrating the regular distribution of a first region and a second region on a body according to an embodiment;

FIG. 2(b) is a schematic diagram illustrating the regular distribution of a first region and a second region on a body according to an embodiment;

FIG. 3 is a schematic comparison diagram of two different light modulators;

FIG. 4 is a schematic structural diagram of an illumination apparatus according to an embodiment;

FIG. 5 is a schematic structural diagram of an illumination apparatus according to an embodiment;

FIG. 6(a) is a schematic structural diagram of a sequencing apparatus according to an embodiment;

FIG. 6(b) is a schematic structural diagram of a sequencing apparatus according to an embodiment;

FIG. 7 is a schematic structural diagram of a sequencing apparatus according to an embodiment;

FIG. 8 is a schematic structural diagram of a sequencing apparatus according to an embodiment;

FIG. 9 is a flowchart of a method of image acquisition according to an embodiment;

FIG. 10 is a schematic diagram illustrating a preset pattern of a square-arranged dot matrix on a chip surface according to an embodiment;

FIG. 11(a) is a schematic diagram of a trajectory involved in one preset movement rule of the square-arranged dot matrix corresponding to FIG. 10;

FIG. 11(b) is a schematic diagram of a trajectory involved in another preset movement rule of the square-arranged dot matrix corresponding to FIG. 10;

FIG. 12 is a schematic diagram illustrating a preset pattern of a triangle-arranged dot matrix on a chip surface according to an embodiment;

FIG. 13(a) is a schematic diagram of a trajectory involved in a preset movement rule of the triangle-arranged dot matrix corresponding to FIG. 12;

FIG. 13(b) is a schematic diagram of a trajectory involved in a preset movement rule of the triangle-arranged dot matrix corresponding to FIG. 12;

FIG. 14 is a schematic diagram illustrating the movement of a stage to switch the field of view according to an embodiment;

FIG. 15(a) is a schematic diagram of a trajectory for updating patterned illumination light and switching the field of view by moving a stage according to an embodiment;

FIG. 15(b) is a schematic diagram of a trajectory for updating patterned illumination light and switching the field of view by moving a stage according to an embodiment;

FIG. 16 is a flowchart of a method for parallel image acquisition and image reconstruction algorithms according to an embodiment;

FIG. 17 is a flowchart of a method for parallel image acquisition and image reconstruction algorithms according to an embodiment;

FIG. 18 is a flowchart of a method for parallel image acquisition and image reconstruction algorithms according to an embodiment;

FIG. 19 is a schematic timing diagram of parallel image acquisition and image reconstruction algorithms according to an embodiment;

FIG. 20 is a flowchart of a method for image reconstruction according to an embodiment;

FIG. 21 is a flowchart for reconstructing an image based on one set of images of a field of view to obtain a reconstructed image of that field of view, according to an embodiment;

FIG. 22 is a flowchart for determining the achievement of iterative convergence according to an embodiment;

FIG. 23 is a flowchart for determining the achievement of iterative convergence according to an embodiment;

FIG. 24 is a schematic diagram illustrating the relationship of the coefficient of variation (CV) value of a reconstructed image and the normalized convergence factor with the number of iterations, according to an embodiment;

FIG. 25 is a flowchart for determining the achievement of iterative convergence according to an embodiment;

FIG. 26 is a schematic diagram illustrating the relationship of the number of iterations and the CV value of a reconstructed image with the step coefficient, according to an embodiment;

FIG. 27 is a flowchart of a method for determining dimensions of a reconstructed image according to an embodiment;

FIG. 28 is a flowchart of a method for image reconstruction according to an embodiment;

FIG. 29 is a schematic diagram of a raw image according to an embodiment;

FIG. 30 is a spectrum diagram of the raw image shown in FIG. 29;

FIG. 31 is a schematic diagram of three frequency dimension values according to an embodiment;

FIG. 32 is a schematic diagram of six frequency dimension ranges according to an embodiment;

FIG. 33 is a schematic diagram of a spectral range of a reconstructed image according to an embodiment;

FIG. 34 is a schematic comparison diagram between raw images and reconstructed images according to an embodiment; and

FIG. 35 is a schematic comparison diagram between raw images and reconstructed images according to an embodiment.

## DETAILED DESCRIPTION

The present disclosure will be illustrated in further detail with reference to the following detailed description and drawings. Like elements in different embodiments have been given like numerals associated therewith. In the following embodiments, numerous specific details are given to provide a thorough understanding of the present application. However, those skilled in the art will readily recognize that some of the features may be omitted or substituted with other elements, materials, and methods in different instances. In some instances, certain operations related to the present application are not illustrated or described in this specification to avoid obscuring the key part of the present application with unnecessary detail. For those skilled in the art, it is not necessary to describe in detail these related operations, as such operations can be fully understood from the description in the specification and the general knowledge in the art. Furthermore, the illustrated features, operations, or characteristics in the specification may be combined in any suitable manner to give various embodiments. Also, the various steps or actions in the description of the methods may be exchanged or adjusted in order, as will be apparent to those skilled in the art. Thus, the various orders in the specification and drawings are for the purpose of clearly illustrating certain embodiments only and are not intended to imply a necessary order unless otherwise indicated that a certain order is necessary.

The serial numbers used herein for the components, such as "first" and "second" are used merely to distinguish between the objects described, and do not carry any sequential or technical meaning. The term "connect" as used herein includes both direct and indirect connections, unless otherwise specified.

The term "sequencing" herein may also be referred to as "nucleic acid sequencing" or "gene sequencing". The three are used interchangeably and refer to the determination of the type and order of bases or nucleotides (including nucleotide analogs) in a nucleic acid molecule.

In some embodiments, the detection of the sample of interest is completed by illuminating the sample of interest with illumination light and then acquiring an image of the sample of interest. Furthermore, the sample of interest emits fluorescence under the excitation of the illumination light, and the detection of the sample of interest is completed by acquiring an image of the sample of interest by means of an optical microscopic imaging system (hereinafter referred to as the "optical system" or "imaging system").

In the embodiments of the present application, the sample of interest may be various samples for which performance or parameter detection is completed or assisted by means of images, including biological samples, such as biological tissues, cells or cell clusters, nucleic acid molecules, enzymes, proteins, or biomolecules; chemical samples, such as materials, mixtures or compositions, organic molecules, inorganic molecules, surfaces containing organic and/or inorganic molecules, or surfaces composed of organic and/or inorganic molecules; as well as other samples that can be detected based on image features, not limited to the listed examples. The "performance or parameter" referred to in the embodiments of the present application includes various indexes involved in the quantitative or qualitative analysis of the sample, including but not limited to: concentration, content, sample composition and/or type, structure, dimension, and the like, but not limited to those listed.

As an example, the sample of interest may be a nucleic acid molecule, and the nucleic acid molecule may be, for example, DNA and/or RNA, or may exist as single-stranded and/or double-stranded forms and/or complexes containing single-stranded or double-stranded nucleic acid sequences. The nucleic acid molecules may be fixed on the surface of a chip. The structure of the chip is not strictly limited. As an example, the chip may have a structure consisting of three layers: an upper layer, a middle layer, and a lower layer, which is also referred to as a sandwich structure. Alternatively, the chip may have a structure consisting of two layers: an upper layer and a lower layer. In some examples, the upper layer is a transparent glass layer, while the middle layer or the lower layer is a transparent or opaque substrate layer. By hollowing out the middle layer or creating indentations on the surface of the lower layer, array-arranged fluid channels can be formed. The fluid channels can hold liquids and provide physical space for reactions. In some examples, a large number of discrete micropores (e.g., one million, ten million, one hundred million, one billion, or more) are arranged on the upper and/or lower surfaces of the fluid channels. The micropores are in circular, rectangular, or other specific shapes. These micropores are arranged in an array on the upper and/or lower surfaces of the fluid channels, forming array patterns, which include but are not limited to triangular, quadrilateral, pentagonal, hexagonal, or octagonal configurations. The diameter of a micropore is generally below 500 nm, and the density of the micropores may be, for example, $10^5$, $10^6$, or $10^7$ per square centimeter. Probes (e.g., oligonucleotides) are fixed inside the micropores, and nucleic acid molecules bind to the probes, for example, by means of hybridization. By adding nucleotides or analogs with optically detectable labels (e.g., fluorophores), polymerases, and the like to the fluid channels, and employing the principle of complementary base pairing, the added nucleotides or analogs bind to the nucleic acid molecules in a complementary pairing manner. The nucleic acid molecules are irradiated with illumination light (or excitation light), which excites the fluorophores on the nucleotides or analogs to generate fluorescence. The fluorescence signal is then acquired by the imaging system to form an image, and finally, base calling is performed based on the image, thereby achieving the determination of the base sequence of the nucleic acid molecules.

In some embodiments, the illumination light is emitted by a light source and can be regulated by a light modulator, allowing the regulated illumination light to irradiate the sample of interest. In the embodiments of the present application, the term "light modulator" refers to an optical element, optical device, or optical sensor configured to regulate the characteristics of light. The characteristics of light include, but are not limited to, the intensity, transmittance, phase, and amplitude of the light.

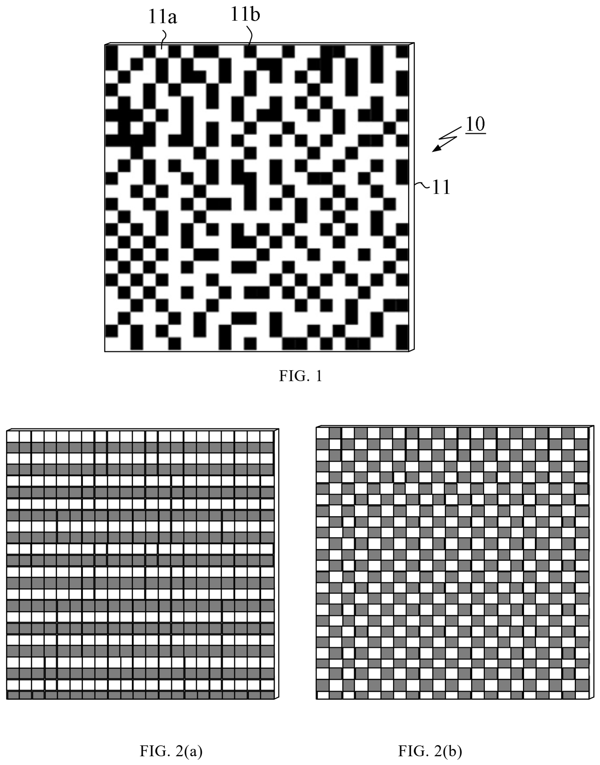

Referring to FIG. 1, some embodiments of the present application provide a light modulator 10. The light modulator 10 includes a body 11, and the body 11 includes a first region 11a and a second region 11b, as specifically described hereinafter.

In some embodiments, the first region 11a has a first light transmittance, and the second region 11b has a second light transmittance. The first light transmittance is different from the second light transmittance.

It should be noted that the term "light transmittance" refers to the degree to which an object allows light to pass through. Light transmittance may be represented as a percentage or a decimal and indicates the extent of light passing through the object. The higher the light transmittance, the greater the extent of light passing through the object; conversely, the lower the light transmittance, the lesser the extent of light passing through the object. Light transmittance directly affects the propagation performance of light.

FIG. 1 illustrates an example. In FIG. 1, the white region represents the first region 11a, and the black region represents the second region 11b. Understandably, FIG. 1 merely illustrates an example and is not intended to limit the number or distribution mode of the first region 11a and the second region 11b.

In some examples, the first light transmittance is greater than a first light transmittance threshold, and the second light transmittance is less than a second light transmittance threshold. The first light transmittance threshold is greater than or equal to the second light transmittance threshold. Thus, by regulating the first light transmittance threshold and the second light transmittance threshold, a certain degree of light transmittance difference can be formed between the first region 11a and the second region 11b. This light transmittance difference causes the light passing through the light modulator 10 to exhibit distinct characteristics, such as light intensity characteristics, thereby forming structured light that meets the desired effect.

In some examples, the first light transmittance is greater than the second light transmittance, and the difference therebetween is greater than a first threshold.

Understandably, the first light transmittance of the first region 11a and the second light transmittance of the second region 11b can be formulated and designed based on actual requirements.

In some embodiments, the first light transmittance is greater than or equal to 70%. For example, the first light transmittance is greater than 70%, 80%, 90%, or 95%. In some embodiments, the second light transmittance is greater than or equal to 30%. For example, the second light transmittance is greater than 30%, 20%, 10%, or 5%.

In some embodiments, the first region 11a is a light-passing region, that is, the first light transmittance is 100%.

In some embodiments, the second region 11b is a light-blocking region, that is, the second light transmittance is 0%.

In one embodiment, the first region 11a has a first refractive index, and the second region 11b has a second refractive index. The first refractive index is different from the second refractive index. Thus, by creating a refractive index difference between the first region 11a and the second region 11b, the light transmittance difference between the two regions can be increased, thereby forming structured light that meets the desired effect. This approach can also diversify the methods for forming structured light.

It should be noted that the refractive index referred to in the embodiments of the present application may be either the relative refractive index or the absolute refractive index. Specifically, when light passes from medium 1 into medium 2 and refraction occurs, the ratio of the sine of the angle of incidence to the sine of the angle of refraction is referred to as the refractive index of medium 2 relative to medium 1, i.e., the "relative refractive index". Meanwhile, the "absolute refractive index" may be understood as the refractive index of a medium relative to a vacuum. The refractive index can also be expressed as a physical quantity representing the ratio of the speed of light in two (isotropic) media.

In some examples, the first refractive index is less than a first refractive index threshold, and the second refractive index is greater than a second refractive index threshold. The first refractive index threshold is less than or equal to the second refractive index threshold.

In some examples, the first refractive index is less than the second refractive index, and the difference therebetween is greater than a second threshold.

Understandably, the first refractive index of the first region 11a and the second refractive index of the second region 11b can be formulated and designed based on actual requirements.

In another embodiment, the first region 11a has a first reflectance ratio, and the second region 11b has a second reflectance ratio. The first reflectance ratio is different from the second reflectance ratio. Thus, by creating a reflectance ratio difference between the first region 11a and the second region 11b, the light transmittance difference between the two regions can be increased, thereby forming structured light that meets the desired effect. This approach can also diversify the methods for forming structured light.

It should be noted that the reflectance ratio of light represents an object's ability to reflect light. This can be defined and calculated as the ratio of the amount of light reflected by the object's surface to the amount of light received thereby, and it can be expressed as a percentage or a decimal.

In some examples, the first reflectance ratio is less than a first reflectance ratio threshold, and the second reflectance ratio is greater than a second reflectance ratio threshold. The first reflectance ratio threshold is less than or equal to the second reflectance ratio threshold.

In some examples, the first reflectance ratio is less than the second reflectance ratio, and the difference therebetween is greater than a third threshold.

Understandably, the first reflectance ratio of the first region 11a and the second reflectance ratio of the second region 11b can be formulated and designed based on actual requirements.

It can be understood that both the refractive index difference and the reflectance ratio difference can simultaneously exist between the first region 11a and the second region 11b.

In one embodiment, the first region 11a has a first thickness, and the second region 11b has a second thickness. The first thickness is different from the second thickness. Thus, through the thickness difference between the first region 11a and the second region 11b, a light transmittance difference can be created between the two regions, thereby forming structured light that meets the desired effect. This approach can also diversify the methods for forming structured light.

In some examples, the first thickness is less than a first thickness threshold, and the second thickness is greater than a second thickness threshold. The first thickness threshold is less than or equal to the second thickness threshold.

In some examples, the first thickness is less than the second thickness, and the difference therebetween is greater than a fourth threshold.

In the embodiments of the present application, there are no strict limitations on the shapes of the first region 11a and the second region 11b on the body 11. In some examples, the shapes of the first region 11a and the second region 11b are identical, such as both being rectangular or triangular. Certainly, it can be understood that the shapes of the first region 11a and the second region 11b may also differ. In some embodiments, the shapes of each first region 11a may even be random, and the shapes of each second region 11b may also be random.

In some embodiments, the body 11 is sheet-like, for example, a rectangular sheet, a square sheet, or a circular sheet.

In some embodiments, a first material is provided on at least one surface of the body 11 to form the first region 11a, and a second material is provided to form the second region 11b. That is, the region of the body 11's surface where the first material is formed is the first region 11a, and the region of the body 11's surface where the second material is formed is the second region 11b.

In some embodiments, the body 11 is a transparent body, and the second material is provided on the transparent body to form the second region 11b, while the regions on the transparent body where the second material is not provided form the first region 11a. In this case, the light transmittance of the second material is lower than that of the transparent body, resulting in a light transmittance difference between the first region 11a and the second region 11b.

In some embodiments, the second material is a material with light transmittance below 50%. In some embodiments, the second material may be a metallic material. Exemplarily, the second material may be metal Cr.

The above provides some descriptions regarding the first region 11a and the second region 11b. In some embodiments, there are a plurality of first regions 11a and a plurality of second regions 11b, that is, the body 11 is provided with a plurality of first regions 11a and a plurality of second regions 11b.

The plurality of first regions 11a and the plurality of second regions 11b are distributed regularly or irregularly. In some embodiments, the plurality of first regions 11a and the plurality of second regions 11b are irregularly distributed on the body 11. Understandably, the term "irregular" here refers to the distribution of the first regions 11a and the second regions 11b on the body 11 lacking any pattern or order.

The following are several examples of regular distributions, which do not fall under the aforementioned irregular distribution scenarios.

Referring to FIG. 2(a), it illustrates Example 1 of a regular distribution: Multiple rows of regions are distributed on the body 11. One row of regions consists entirely of the first regions 11a, the next row of regions consists entirely of the second regions 11b, and the subsequent row of regions again consists entirely of the first regions 11a, alternating in this manner. In the figure, the white-filled squares represent the first regions 11a, and the gray-filled squares represent the second regions 11b.

Referring to FIG. 2(b), it illustrates Example 2 of a regular distribution: Multiple rows of regions are distributed on the body 11. In each row of regions, the first regions 11a and the second regions 11b are arranged alternately. In the figure, the white-filled squares represent the first regions 11a, and the gray-filled squares represent the second regions 11b.

In some embodiments, a plurality of first regions 11a and a plurality of second regions 11b are randomly distributed on the body 11. During the design process, the positions of the first regions 11a and the second regions 11b on the body 11 may be calculated using a random algorithm.

In some embodiments, the total area of the plurality of first regions 11a and the total area of the plurality of second regions 11b have a ratio ranging from 1:3 to 3:1. That is, the total area of all first regions 11a and the total area of all second regions 11b on the body 11 have a ratio ranging from 1:3 to 3:1. Furthermore, the ratio between the two is 1:1, that is, the total areas of the two are equal.

In some embodiments, the surface of the body 11 can be divided into a plurality of unit areas. Within the region of any unit area, first regions 11a in a first quantity range are randomly distributed. For example, the first quantity range may be 120 to 1500.

In some embodiments, the surface of the body 11 can be divided into a plurality of unit areas. Within the region of any unit area, second regions 11b in a second quantity range are randomly distributed. For example, the second quantity range may be 120 to 1500.

It should be noted that the shape of the unit area may be square, rectangular, circular, triangular, or other shapes. The unit for the unit area may be, for example, square micrometers.

In some embodiments, by arranging a plurality of first regions 11a and a plurality of second regions 11b on corresponding positions of the body 11 in an irregular or even random manner, the plurality of first regions and the plurality of second regions are distributed on the body 11 in a manner where the optical modulation degree is greater than a fifth threshold. That is, the positions of the plurality of first regions 11a and second regions 11b provided on the body 11 cause the optical modulation degree to be greater than the fifth threshold.

In the embodiments of the present application, the optical modulation degree is equal to the ratio of the difference in light intensity to the sum of light intensity within a selected region or range. The difference in light intensity is the difference between the maximum light intensity and the minimum light intensity, and the sum of light intensity is the sum of the maximum light intensity and the minimum light intensity.

In one embodiment, when the selected region is the body 11, the maximum light intensity is the maximum light intensity within the range of the body 11, and the minimum light intensity is the minimum light intensity within the range of the body 11. In another embodiment, when the selected region is a partial region of the body 11, the maximum light intensity is the maximum light intensity within the selected region of the body 11, and the minimum light intensity is the minimum light intensity within the selected region of the body 11. The shape of the selected region is not limited and may include regions corresponding to strip shapes or regions corresponding to various other shapes. When the shape of the selected region is a strip shape, the width of the strip can be sufficiently narrow, such that the corresponding strip appears as a line. It should be understood that the line may be a straight line, a curved line, or even a randomly formed line.

As an example, the optical modulation degree is equal to the ratio of the difference in light intensity to the sum of light intensity for a specified row of the body 11. The difference in light intensity is the difference between the maximum light intensity and the minimum light intensity, and the sum of light intensity is the sum of the maximum light intensity and the minimum light intensity. The maximum light intensity is the maximum light intensity within the region corresponding to the specified row, and the minimum light intensity is the minimum light intensity within the region corresponding to the specified row.

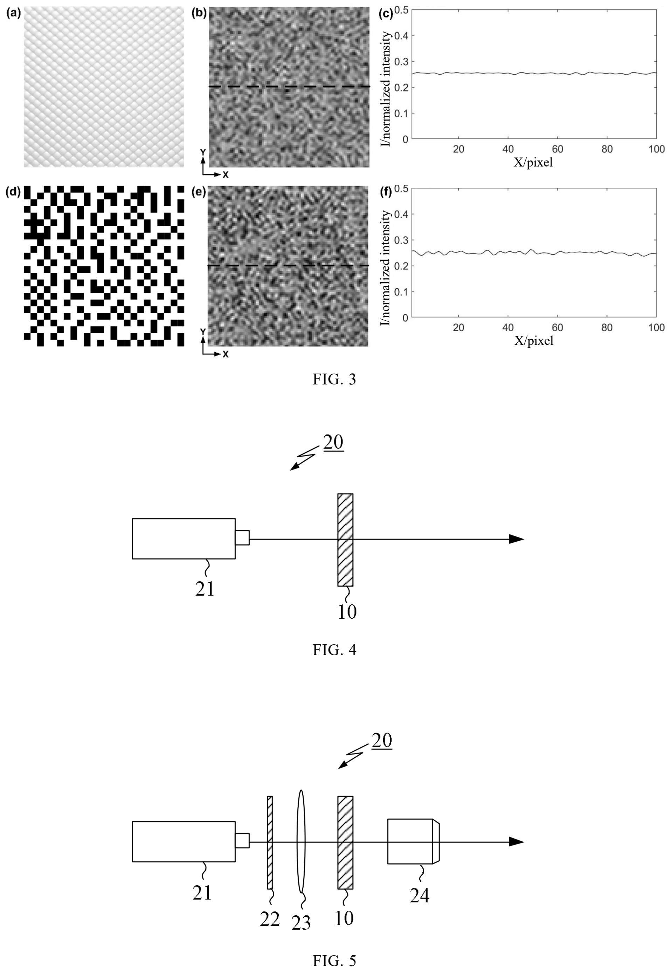

FIG. 3 is a schematic comparison diagram of a light modulator 10 according to some embodiments of the present application and a conventional light modulator. In the figure, (a) illustrates a schematic view of a conventional light modulator, such as a microlens array; (b) illustrates a patterned illumination light obtained after the illumination light passes through the microlens array shown in (a); (c) illustrates the light intensity of a specified row, marked with a dashed line in (b); (d) illustrates a schematic view of the light modulator 10 according to an embodiment of the present application; (e) illustrates a patterned illumination light obtained after the illumination light passes through the light modulator 10 shown in (d); and (f) illustrates the light intensity of a specified row, marked with a dashed line in (e).

In the embodiments of the present application, the calculation formulas for the maximum light intensity $I_{max}$ and the minimum light intensity $I_{min}$ are as follows:

$$I_{max} = I_0 \cdot (1 + m)$$

$$I_{min} = I_0 \cdot (1 - m)$$

Accordingly, the calculation formula for the optical modulation degree m is as follows:

$$m = \frac{I_{max} - I_{min}}{I_{max} + I_{min}}$$

For the conventional light modulator, such as the microlens array shown in FIG. (a), the normalized maximum and minimum light intensities in (c) are 0.2586 and 0.2482, respectively. Therefore, the optical modulation degree is: m = 0.0205.

For the light modulator 10 according to an embodiment of the present application as shown in FIG. (d), the normalized maximum and minimum light intensities in (f) are 0.2626 and 0.2365, respectively. Therefore, the optical modulation degree is: m = 0.0523.

Therefore, the light modulator 10 according to an embodiment of the present application as shown in FIG. (d) achieves an optical modulation degree far higher than that of the conventional light modulator, such as the microlens array shown in FIG. (a), improving the optical modulation degree to $\frac{0.0523}{0.0205} * 100\% = 255.12\%$ the original.

The above provides some descriptions regarding the light modulator 10. The light modulator 10 modulates the illumination light to produce patterned illumination light, which exhibits a higher optical modulation degree.

Referring to FIG. 4, in some embodiments of the present application, an illumination apparatus 20 is further disclosed. The illumination apparatus 20 includes a light source 21 and the light modulator 10 as described in any one of the embodiments herein. Referring to FIG. 5, in some embodiments, the illumination apparatus 20 further includes at least one of a filter 22, an aspherical lens 23, and an objective lens 24. It should be noted that the solid arrows in FIGS. 4 and 5 indicate the optical path.

In some embodiments, the light source 21 is configured to emit illumination light. The light source 21 may be implemented using an LED, laser, or the like.

In some embodiments, the filter 22 is located on the optical path of the illumination light and is configured to filter the illumination light.

In some embodiments, the aspherical lens 23 is located on the optical path of the illumination light and is configured to control the beam aperture of the illumination light entering the objective lens 24.

In some embodiments, the objective lens 24 is located on the optical path of the illumination light and is configured to project the illumination light onto a sample of interest.

In some embodiments, the filter 22, the aspherical lens 23, the light modulator 10, and the objective lens 24 are sequentially distributed along the optical path of the illumination light.

In some embodiments, the light modulator 10 has a first region 11a with a first diameter length of A1 and a second region 11b with a second diameter length of A2, where $A1/K \geq r$ and $A2/K \geq r$, with K representing the reduction factor of the light beam after passing through the objective lens 24, and r representing the diffraction limit resolution of the objective lens 24.

It should be noted that the first diameter length A1 is used to measure the size of the first region 11a. When the first region 11a is rectangular, the first diameter length A1 refers to the length of the longer side or the shorter side of the first region 11a. When the first region 11a is square, the first diameter length A1 refers to the length of a side of the first region 11a. When the first region 11a is triangular, the first diameter length A1 refers to the length of the longest side of the first region 11a. When the first region 11a is circular, the first diameter length A1 refers to the diameter length of the first region 11a. Similarly, the second diameter length A2 is used to measure the size of the second region 11b. When the second region 11b is rectangular, the second diameter length A2 refers to the length of the longer side or the shorter side of the second region 11b. When the second region 11b is square, the second diameter length A2 refers to the length of a side of the second region 11b. When the second region 11b is triangular, the second diameter length A2 refers to the length of the longest side of the second region 11b. When the second region 11b is circular, the second diameter length A2 refers to the diameter of the second region 11b.

The illumination apparatus 20 according to some embodiments of the present application can be applied to gene sequencing, where the illumination light is projected onto the sample of interest to excite the sample of interest to generate fluorescence.

The introduction of the light modulator 10 into the illumination apparatus 20 enables the illumination light projected onto the sample of interest to exhibit a higher optical modulation degree, such that the image reconstructed based on the acquired raw image has a better reconstruction effect.

The introduction of the light modulator 10 into the illumination apparatus 20 enables the illumination light projected onto the sample of interest to exhibit a higher optical modulation degree, thereby reducing the number of raw images required to be acquired for the reconstructed image.

By introducing the aspherical lens 23 into the illumination apparatus 20, the aperture of the illumination light entering the objective lens 24 can be controlled. This can be achieved by adjusting the distance between the aspherical lens 23 and the objective lens 24, ensuring that the illumination beam uniformly illuminates the entire field of view and better excites the sample of interest to generate fluorescence.

In some embodiments where the illumination apparatus 20 is applied to gene sequencing, the light source 21 can be configured to emit illumination light with a wavelength of $\lambda 1$ and a wavelength of $\lambda 2$. Each type of light can excite the fluorophores of two bases, achieving sequencing of the four bases A, T, G, and C.

The above provides some descriptions regarding the illumination apparatus 20. Through the illumination apparatus 20, patterned illumination light can be obtained, and this patterned illumination light exhibits a higher optical modulation degree.

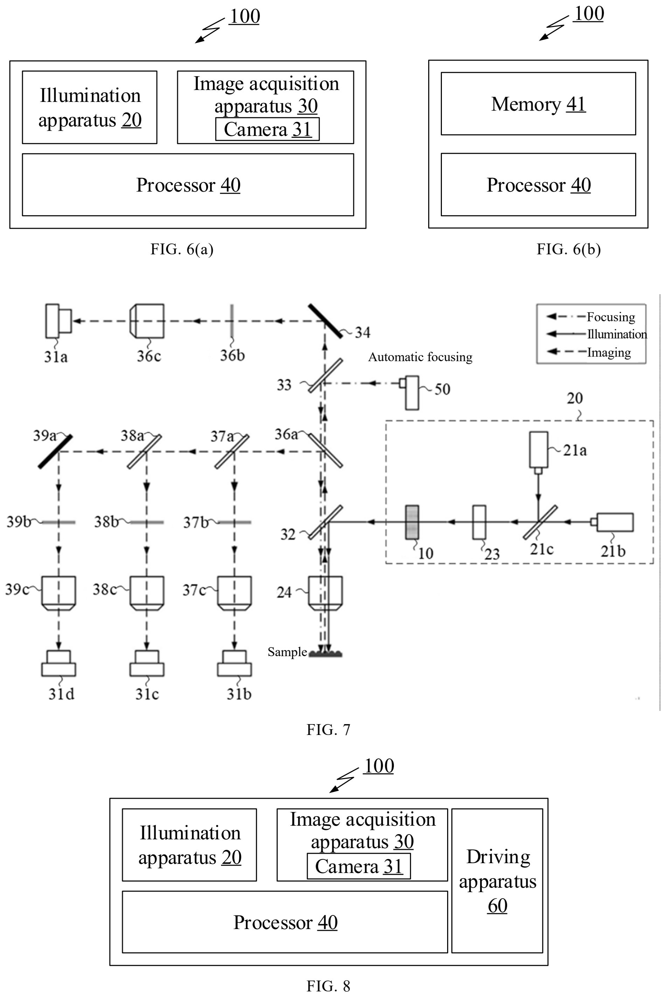

Referring to FIG. 6(a) or 6(b), in some embodiments of the present application, a sequencing apparatus 100 is further disclosed. The sequencing apparatus 100 is configured to perform gene sequencing on a sample of interest, for example, by projecting illumination light onto the sample of interest, such that the sample of interest is excited to generate fluorescence, acquiring a raw image, and performing image reconstruction on the raw image.

In some embodiments, the sequencing apparatus 100 includes an illumination apparatus 20. In some embodiments, the sequencing apparatus 100 further includes an image acquisition apparatus 30 and a processor 40.

In some embodiments, the sequencing apparatus 100 includes the processor 40 and a memory 41.

In some embodiments, the sequencing apparatus 100 includes the illumination apparatus 20, the image acquisition apparatus 30, the processor 40, and the memory 41.

Each component is explained hereinafter.

In some embodiments, the illumination apparatus 20 is configured to project illumination light onto the sample of interest, causing the sample of interest to be excited and generate fluorescence. The image acquisition apparatus 30 acquires a raw image, and the processor 40 is configured to perform image reconstruction on the raw image. In some embodiments, the illumination apparatus 20 may be the illumination apparatus 20 described in any one of the above embodiments, which will not be repeated here. In some embodiments, the image acquisition apparatus 30 includes a camera 31.

In some embodiments, the memory 41 is configured to store programs. In some embodiments, the memory 41 is configured to store data, such as the raw image mentioned herein or the reconstructed image (i.e., post-reconstruction image) mentioned herein.

In some embodiments, the processor 40 can be implemented using one or more of the following: circuits, a single or a plurality of application specific integrated circuits (ASIC), a digital signal processor (DSP), a digital signal processing device (DSPD), a programmable logic device (PLD), a field programmable gate array (FPGA), a central processing unit (CPU), or a graphic processing unit (GPU). For example, the processor 40 may include a GPU. For another example, the processor 40 may include both a CPU and a GPU.

In some embodiments, the processor 40 can control some components to operate.

In some embodiments, the processor 40 can implement the method described in any one of the embodiments herein, or some or all of the steps in the method by running the program in the memory 41.

FIG. 7 illustrates an example of an optical system.

In FIG. 7, the optical path of the illumination light (represented by solid-line arrows) from the illumination apparatus 20 is mainly used to provide illumination light. In embodiments containing a light modulator 10, the light modulator 10 modulates the illumination light so that the light projected onto the sample of interest becomes a patterned illumination light. The illumination apparatus 20 is configured to excite the sample of interest to generate a fluorescence signal. The light source 21 can adopt a structure from the prior art or the structure shown in FIG. 7. In the structure shown in FIG. 7, the light source 21 includes a light source 21a with an excitation wavelength of $\lambda1$ and a light source 21b with an excitation wavelength of $\lambda2$. A dual-wavelength light source 21 is used to excite fluorophores on different bases, thereby enabling sequencing of the four bases A, T, G, and C. Specifically, the dual-wavelength light source 21 achieves beam combination via a dichroic mirror 21c. Afterward, the beam is expanded by an aspherical lens 23 or the like, and the illumination light field of the expanded light beam is modulated by the light modulator 10 to generate patterned illumination light. This enables the image acquisition apparatus 30 to acquire an image formed by the fluorescence signal, thereby obtaining a raw image.

The objective lens 24 is arranged on the optical path of the illumination and is configured to project the patterned illumination light onto the sample of interest, which is placed on the stage 61, and to receive the fluorescence signal generated by the sample of interest. The camera 31 is configured to receive the fluorescence signal and acquire a raw image.

The generated patterned illumination light is projected onto the surface of the sample through the objective lens 24. The excited fluorescence signal is also received by the objective lens 24 and subsequently passes through a dichroic mirror group (including a first dichroic mirror 36a, a second dichroic mirror 37a, a third dichroic mirror 38a, a fourth dichroic mirror 33, and a fifth dichroic mirror 32), a reflector group (including a first reflector 39a and a second reflector 34), and a filter group (including a first filter 36b, a second filter 37b, a third filter 38b, and a fourth filter 39b). The fluorescence signals corresponding to the four bases A, T, G, and C are filtered out respectively and enter the four cameras 31 to achieve random illumination wide-field fluorescence imaging, obtaining a random illumination scanned wide-field image. The fifth dichroic mirror 32 is arranged on the optical path of the illumination light and is configured to reflect the patterned illumination light to the objective lens 24, so that the patterned illumination light, after passing through the objective lens 24, illuminates the sample of interest to excite the sample of interest to generate fluorescence of at least one wavelength. For example, the fifth dichroic mirror 32 is arranged along the optical axis of the objective lens 24 and placed at a 45° angle with respect to the optical axis of the objective lens 24. The fifth dichroic mirror 32 is further configured to transmit the fluorescence received by the objective lens 24 into the beam-splitting mechanism, which will be further explained hereinafter.

The image acquisition apparatus 30 collects multi-band fluorescence signals and forms images through the imaging optical path (indicated by dashed arrows). The image acquisition apparatus 30 can adopt the specific structure in the prior art or the structure shown in FIG. 7. The image acquisition apparatus 30 further includes a beam-splitting mechanism and can reuse the fifth dichroic mirror 32. The fifth dichroic mirror 32 is further configured to transmit the fluorescence received by the objective lens 24 into the beam-splitting mechanism, such that different light beams ultimately enter different cameras 31.

For the acquisition of fluorescence signals corresponding to the four bases A, T, G, and C in the sample of interest, the optical path structure, as shown in FIG. 7, can be adopted. The image acquisition apparatus 30 accordingly includes four cameras, for example, a first camera 31a, a second camera 31b, a third camera 31c, and a fourth camera 31d.

The first dichroic mirror 36a is configured to split the fluorescence transmitted through the fifth dichroic mirror 32, forming a first beam and a second beam. The first dichroic mirror 36a allows the transmission of the first beam so that the first beam is received by the first camera 31a after passing through a first filter 36b and a first tube lens 36c. The first dichroic mirror 36a reflects the second beam to the second dichroic mirror 37a. Specifically, the first dichroic mirror 36a is arranged along the optical axis of the objective lens 24, and taking the plane where the fifth dichroic mirror 32 is located as the reference plane, the first dichroic mirror 36a is rotated 90° counterclockwise and arranged in this orientation. The fluorescence transmitted through the fifth dichroic mirror 32 is split by the first dichroic mirror 36a, forming the first beam and the second beam. The first beam, after the transmission through the first dichroic mirror 36a, passes through the first filter 36b and the first tube lens 36c and is then received by the first camera 31a. Both the first filter 36b and the first tube lens 36c are arranged perpendicular to the optical axis. The second beam is reflected by the first dichroic mirror 36a to the second dichroic mirror 37a.

To make full use of the spatial structure, a second reflector 34 can be added to the optical path of the first beam to change the direction of the first beam. The first beam is then reflected to the first filter 36b and, after passing through the first tube lens 36c, is received by the first camera 31a.

The second dichroic mirror 37a is configured to transmit part of the second beam to form a third beam, and to reflect part of the second beam through a second filter 37b and a second tube lens 37c, allowing part of the second beam to be received by the second camera 31b. Specifically, the second dichroic mirror 37a is arranged along the optical axis of the second beam and is arranged parallel to the plane where the fifth dichroic mirror 32 is located, using the plane as a reference plane; the second dichroic mirror 37a is configured to transmit part of the second beam to form the third beam and reflect part of the second beam to the second filter 37b. Both the second filter 37b and the second tube lens 37c are arranged along the optical axis of the reflected part of the second beam, as well as perpendicular to the optical axis of the reflected part of the second beam. The part of the second beam filtered by the second filter 37b is received by the second camera 31b after passing through the second tube lens 37c.

The third dichroic mirror 38a is configured to transmit part of the third beam to form a fourth beam, and to reflect part of the third beam through a third filter 38b and a third tube lens 38c, allowing part of the third beam to be received by the third camera 31c. Specifically, the third dichroic mirror 38a is arranged along the optical axis of the third beam and is arranged parallel to the plane where the fifth dichroic mirror 32 is located, using the plane as a reference plane; the third dichroic mirror 38a is configured to transmit part of the third beam to form the fourth beam and reflect part of the third beam to the third filter 38b. Both the third filter 38b and the third tube lens 38c are arranged along the optical axis of the reflected third beam, as well as perpendicular to the optical axis of the reflected third beam. The part of the third beam filtered by the third filter 38b is received by the third camera 31c after passing through the third tube lens 38c.

The first reflector 39a is configured to reflect a fourth beam so that the fourth beam passes through a fourth filter 39b and a fourth tube lens 39c and is then received by the fourth camera 31d. Specifically, the first reflector 39a is arranged along the optical axis of the fourth beam and is arranged parallel to the plane where the fifth dichroic mirror 32 is located, using the plane as a reference plane; the first reflector 39a is configured to reflect the fourth beam to the fourth filter 39b. Both the fourth filter 39b and the fourth tube lens 39c are arranged along the optical axis of the reflected fourth beam, as well as perpendicular to the optical axis of the reflected fourth beam. The fourth beam filtered by the fourth filter 39b is received by the fourth camera 31d after passing through the fourth tube lens 39c.

By adopting the above optical path arrangement method, it is possible to simultaneously acquire fluorescence signals corresponding to the four bases A, T, G, and C, while ensuring super-resolution imaging precision and improving the spatial utilization of the imaging optical path system.

In some embodiments, the sequencing apparatus 100 further includes a focusing assembly 50. The dash-dotted arrows in the figure represent the optical path of the focusing assembly 50, and the focusing assembly 50 is mainly configured to achieve automatic focusing, thereby facilitating the acquisition of clear images within the range of depth of focus. The fourth dichroic mirror 33 is configured to reflect the focusing beam emitted by the focusing assembly 50 to the objective lens 24 and to transmit the aforementioned first beam. The fourth dichroic mirror 33 is further configured to reflect the focusing beam reflected from the surface of the sample of interest to the focusing assembly 50 to achieve focus detection.

Referring to FIG. 8, in some embodiments, the sequencing apparatus 100 further includes a driving apparatus 60.

In some embodiments, the driving apparatus 60 is configured to drive the light modulator 10 to move, such as driving the light modulator 10 to move relative to the illumination light, or, for another example, driving the light modulator 10 to move relative to the objective lens 24.

In some embodiments, the driving apparatus 60 is configured to drive the stage 61 to move. The stage 61 is configured to carry the sample of interest.

The driving apparatus 60 can be implemented, for example, using existing structures or structures that may emerge in the future, which is not limited herein. For example, the driving apparatus 60 can be implemented using a sliding stage structure or the like.

The above provides some descriptions regarding the sequencing apparatus 100.

In some embodiments of the present application, a method of image acquisition is disclosed. In some embodiments, the method for image reconstruction and the method of image acquisition can be applied to the sequencing apparatus 100 according to some embodiments herein. In other words, the image acquisition provided according to the embodiments of the present application can be implemented with the sequencing apparatus 100 described above. It should be understood that the image acquisition provided hereinafter in the present application can also be carried out using other image acquisition apparatuses different from the sequencing apparatus 100 described above.

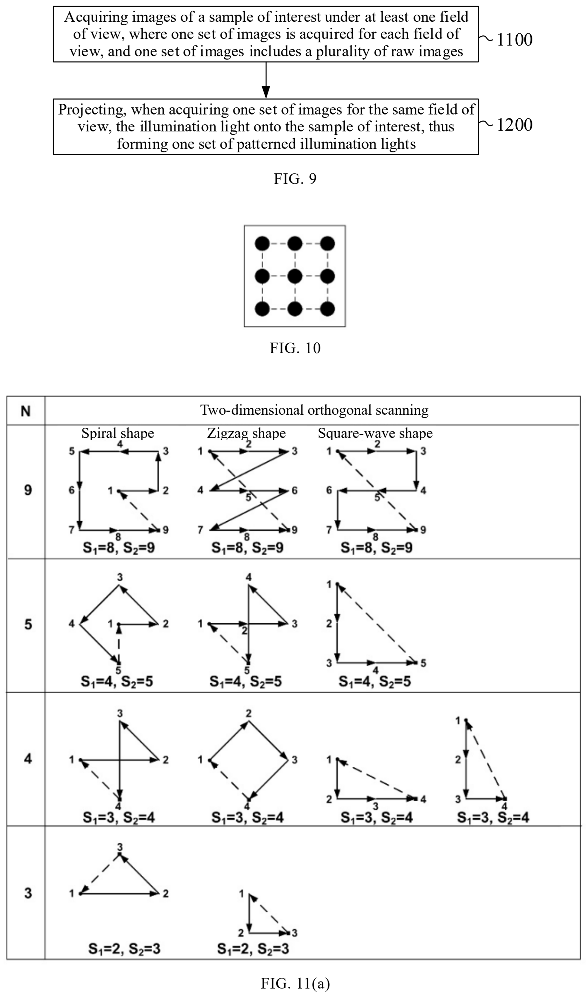

Referring to FIG. 9, in some embodiments, the method of image acquisition includes the following steps.

In step 1100, images of a sample of interest are acquired under at least one field of view. One set of images is acquired for each field of view, and one set of images includes a plurality of raw images.

The field of view (FOV) is a concept in the optical imaging field, which can be directly understood as the region observable by the image acquisition apparatus, such as a camera, during the optical imaging (photographing) of a target object by an imaging system, that is, the visible region or the scope of vision, i.e., the field of view.

In some implementations, when performing optical imaging on the sample of interest, a single field of view may not cover the entire area of the sample of interest. Therefore, when performing optical imaging on the sample of interest, the sample of interest is divided into a plurality of fields of view for image acquisition. Specifically, the image acquisition apparatus, after acquiring the raw images of one field of view of the sample of interest, switches to the next field of view of the sample of interest and continues acquiring raw images, until raw images of all fields of view of the sample of interest are acquired.

In one implementation, different regions of the sample of interest can be imaged, for example, by driving the movement of the stage 61 via a driving apparatus 60, thereby enabling the switching of fields of view, that is, switching from one field of view to the next field of view.

In the embodiments of the present application, during image acquisition for at least one field of view of the sample of interest, one set of images is acquired for each field of view. It should be understood that the one set of images corresponds to images obtained by performing multiple image acquisitions of the sample of interest under the same field of view, but this does not imply that the acquired objects, or the acquired images, are completely identical. It can be understood that one set of images may be derived from imaging part of the regions in the sample of interest under the same field of view, and each time when the sample of interest under the same field of view is imaged, the part of the regions acquired in the sample of interest is different. In other words, one set of images may be derived from imaging different shapes or patterns presented by the sample of interest under the same field of view. Thus, the images within one set of acquired images are all different from each other.

In the embodiments of the present application, the sample of interest may be a sample that inherently has a certain solid-phase shape, such as a surface to be tested, or a sample that requires fixation on another carrier, such as a chip, like nucleic acid molecules. The present application does not impose limitations in this regard.

In the embodiments of the present application, one set of images acquired from a sample of interest under the same field of view are all raw images. That is, one set of images includes a plurality of raw images, or, one set of images acquired from the sample of interest under the same field of view consists of a plurality of raw images. It should be understood that the term "raw image" as referred to in the embodiments of the present application refers to the unprocessed images obtained after imaging by the image acquisition apparatus.

In the embodiments of the present application, the different shapes or patterns presented by the sample of interest under the same field of view can be achieved using various methods. In one possible embodiment, when acquiring one set of images for the same field of view, the illumination light is projected onto the sample of interest, forming one set of patterned illumination lights. In this case, each patterned illumination light is projected (or cast) onto the sample of interest under the same field of view, causing the sample of interest to present different patterns within the field of view. By performing optical image acquisition for each pattern, one set of images derived from the same field of view of the sample of interest can be obtained.

In some embodiments, for the same field of view, one set of patterned illumination lights includes a plurality of different patterned illumination lights, and the plurality of different patterned illumination lights correspond one-to-one with the plurality of raw images included in one set of images for that field of view.

That is, for any one of the fields of view of the sample of interest (referred to here as field of view F), one set of patterned illumination lights for the field of view F includes a plurality of different patterned illumination lights. Moreover, under the current field of view, one raw image is acquired separately for the sample of interest under the illumination of each patterned illumination light, such that the plurality of different patterned illumination lights correspond one-to-one with the plurality of raw images included in one set of images for the field of view F. For the field of view F, by projecting the patterned illumination light onto the sample of interest, a plurality of raw images are acquired in the field of view F. In some embodiments, during the acquisition of the plurality of raw images for the field of view F, one set of patterned illumination lights for the field of view F are sequentially projected (or cast) onto the sample of interest in temporal order. After one patterned illumination light from the set is projected onto the sample of interest, one raw image of the field of view F is acquired. Then, the next patterned illumination light from the set is projected onto the sample of interest, and the next raw image of the field of view F is acquired. This process is repeated until the last patterned illumination light from the set is projected onto the sample of interest, and the last raw image of the field of view F is acquired. At this point, the acquisition of all raw images for the field of view F is complete. Subsequently, the sample of interest is switched from the field of view F to the next field of view. In the next field of view, the acquisition of all raw images for this field of view continues. This process is repeated until the last field of view. As described above, the switching of fields of view can be achieved, for example, by driving the movement of the stage 61 via a driving apparatus 60, that is, switching from one field of view to the next field of view.

The embodiments of the present application involve patterned illumination light as well as the switching, updating, or variation of the patterned illumination light. This will be explained in detail hereinafter.

In one embodiment, when acquiring one set of images for the same field of view, the illumination light is modulated by a light modulator and is projected onto the sample of interest, forming one set of patterned illumination lights within the field of view. In some embodiments, the light modulator may be the light modulator 10 disclosed in some embodiments above. For example, the light modulator 10 includes a body 11, and the body 11 includes a plurality of first regions 11a and a plurality of second regions 11b randomly distributed on the body 11. The first region 11a has a first light transmittance, and the second region 11b has a second light transmittance. The first light transmittance is different from the second light transmittance. To save space, the composition, types, and optional configurations of the light modulator 11 will not be repeated here.

In some embodiments, the patterned illumination light projected by the light modulator 10 is a random patterned illumination light. That is, each of the patterned illumination lights constituting one set of patterned illumination lights is randomly presented without strict regularity. Compared to methods that require mechanical or manual adjustments to the projected illumination light patterns, under the premise of achieving the same resolution effect, the embodiments of the present application use the light modulator 10 to generate random patterned illumination light, which can reduce the number of image acquisitions.

In the embodiments of the present application, by reconstructing one set of images derived from the same field of view, the reconstructed image obtained can serve as the output image for that field of view, such as a super-resolution image. In the present application, when acquiring one set of images for the same field of view, the illumination light is modulated by a light modulator 10 and is projected onto the sample of interest, forming one set of patterned illumination light within the field of view. Since the light modulator 10 exhibits a higher optical modulation degree and enables good randomness in one set of patterned illumination lights, the number of raw images required to be acquired for the same field of view can be reduced. For example, the number is reduced to between 2 and 9 raw images while still achieving good super-resolution effects.

In addition, in some embodiments, for any one of the fields of view of the sample of interest (referred to here as field of view F), during the acquisition of a plurality of raw images for the field of view F, the pattern of the illumination light projected onto the sample of interest in the field of view F is altered by adjusting the light modulator 10. Each time the patterned illumination light is changed, a corresponding raw image is acquired. By designing a preset movement rule, the number of raw images required to be acquired for the same field of view can be reduced. For example, the number is reduced to between 2 and 9 raw images, while still achieving good super-resolution effects.

A detailed explanation is provided hereinafter.

In some embodiments, when acquiring one set of images for the same field of view, the light modulator 10 is controlled to move according to the preset movement rule in order to change the patterned illumination light projected onto the field of view, thereby resulting in one set of patterned illumination lights for the field of view.

In some embodiments, each time the light modulator 10 moves, the pattern of the illumination light changes once, i.e., the formed patterned illumination light changes once. Therefore, each raw image for the same field of view is acquired under a different patterned illumination light.

Understandably, taking any one of the fields of view of the sample of interest (referred to here as field of view F) as an example, the light modulator 10 is moved according to a preset movement rule, and with each movement, the patterned illumination light projected onto the field of view F is changed or updated once, while one raw image of the field of view F is acquired. After the last raw image of the field of view F is acquired, the sample of interest is switched from the field of view F to the next field of view by, for example, driving the stage 61 to move, and one set of raw images of the next field of view is continuously acquired until the last field of view.

The above provides some explanation regarding changing the patterned illumination light projected (or cast) onto the field of view F by controlling the movement of the light modulator 10 and switching fields of view by controlling the movement of the stage 61.

In another embodiment, when acquiring one set of images for the same field of view, the patterned illumination light projected onto the field of view is changed by controlling the sample of interest to move according to a preset movement rule, thereby resulting in one set of patterned illumination lights for the field of view. It should be understood that in this embodiment, the sample of interest moves according to a preset movement rule, causing the pattern of the illumination light projected onto the sample of interest within the current field of view to change, without changing the scope of the field of view. The movement of the sample of interest can be implemented by adjusting the angle or tilt of the sample of interest relative to the optical path.

In some embodiments, each time the sample of interest moves, the pattern of the illumination light changes once, i.e., the formed patterned illumination light changes once. Therefore, each raw image for the same field of view is acquired under a different patterned illumination light. Understandably, taking any one of the fields of view of the sample of interest (referred to here as field of view F) as an example, the sample of interest is moved according to a preset movement rule (e.g., by driving the stage 61 to move the sample of interest), and with each movement, the patterned illumination light projected onto the field of view F is changed or updated once, while one raw image of the field of view F is acquired. After the last raw image of the field of view F is acquired, the sample of interest is switched from the field of view F to the next field of view by, for example, driving the stage 61 to move, and one set of raw images of the next field of view is continuously acquired until the last field of view.

Therefore, switching fields of view can be achieved, for example, by driving the movement of the stage 61 via a driving apparatus 60, i.e., switching from one field of view to the next field of view; in addition, the patterned illumination light projected (or cast) onto the field of view F can be altered by controlling the movement of the stage 61. Therefore, throughout the process, the light modulator 10 does not need to move but remains in a stationary state. The entire process only requires moving the stage 61, which allows the reuse of the structure originally used to move the stage 61 for field-of-view switching. This simplifies the overall structure, streamlines the timing sequence, and reduces structural costs.

Whether by controlling the light modulator 10 to move according to a preset movement rule to change the patterned illumination light projected onto the field of view or by controlling the sample of interest to move according to a preset movement rule to change the patterned illumination light projected onto the field of view, a preset movement rule is involved. The following provides an explanation of the preset movement rule.

In yet another embodiment, when acquiring one set of images for the same field of view, the patterned illumination light projected onto the field of view can be changed by simultaneously controlling both the light modulator 10 and the sample of interest to move according to a preset movement rule, thereby resulting in one set of patterned illumination lights for the field of view. In some embodiments, the preset movement rule is determined by a preset pattern of the sample of interest on the surface of the chip. Thus, more image details can be obtained by combining the pattern of the sample of interest on the surface of the chip, thus enriching the acquired images. It can be understood that the chip is a carrier, such as a substrate or baseplate, for carrying the sample of interest. Exemplarily, the sample of interest is arranged in a dot matrix on the surface of the chip, forming a fixed preset pattern. When the sample of interest itself forms a surface in a certain solid state, the preset pattern of the sample of interest on the surface of the chip may also be understood as the preset pattern of the sample of interest on the surface.

In some embodiments, the preset movement rule includes a trajectory composed of one or more step paths.

In some embodiments, when the preset pattern on the surface of the chip is a quadrilateral-arranged dot matrix, the preset movement rule includes the following: the turning points of the trajectory as a whole form a quadrilateral or a triangle; or the trajectory is parallel to one side of the quadrilateral.

FIG. 10 illustrates an example in which the preset pattern on the surface of the chip is a square-arranged dot matrix. Two points connected in the same row, together with two points in the same column as the above two points in an adjacent row, form a square. Four squares, with one of the corners as the center, form a large square as shown in FIG. 10.

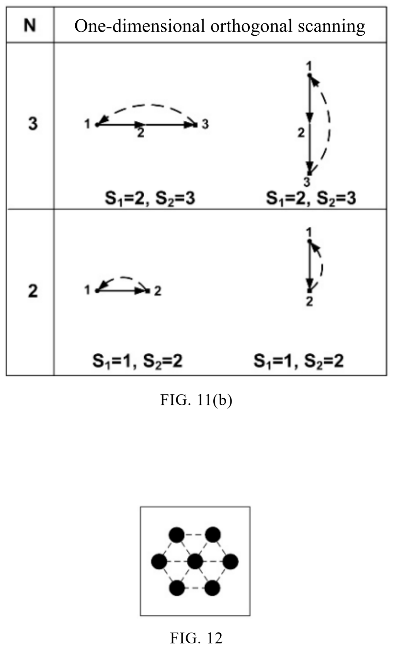

In conjunction with FIGS. 11(a) and 11(b), the trajectory involved in the preset movement rule is explained hereinafter. It should be noted that in the figure, N represents the number of nodes in the trajectory. Understandably, two adjacent nodes can define a one-step path. The sequence number of each node on the trajectories is also marked in the figure, and the movement direction is indicated by arrows on the trajectories.

In some examples, when the preset pattern on the surface of the chip is a quadrilateral-arranged dot matrix, the preset movement rule includes the following: the turning points of the trajectory as a whole form a quadrilateral or a triangle. In some examples, the turning points of the trajectory as a whole forming a quadrilateral or a triangle includes any of the following:

(1) The nodes of the trajectory include the center of the quadrilateral and the nodes located on each side of the quadrilateral. Starting from the center of the quadrilateral, the trajectory reaches a node on a side of the quadrilateral, and then, from that node, traverses each node on the sides of the quadrilateral in one direction, or vice versa. The one direction is a clockwise direction or a counterclockwise direction.

For example, the first trajectory diagram in FIG. 11(a) where N = 9 is an example.

(2) A Z-shaped or zigzag-shaped trajectory.

For example, the second trajectory diagram in FIG. 11(a) where N = 9 is an example.

(3) A square-wave-shaped trajectory.

For example, the third trajectory diagram in FIG. 11(a) where N = 9 is an example.

(4) The nodes of the trajectory include the four vertices of the quadrilateral. Starting from one vertex of the quadrilateral, the trajectory reaches the opposite vertex, then to the adjacent vertex of the opposite vertex, and then to the opposite vertex of that adjacent vertex.

For example, the first trajectory diagram in FIG. 11(a) where N = 4 is an example.

(5) The nodes of the trajectory include the center and four vertices of the quadrilateral. Starting from one vertex of the quadrilateral, the trajectory passes through the center to the opposite vertex, then to the adjacent vertex of the opposite vertex, and then to the opposite vertex of the adjacent vertex.

For example, the second trajectory diagram in FIG. 11(a) where N = 5 is an example.

(6) The nodes of the trajectory include the center and four vertices of the quadrilateral. Starting from the center of the quadrilateral, the trajectory reaches one vertex of the quadrilateral and then traverses each vertex on the sides of the quadrilateral in one direction. The one direction is a clockwise direction or a counterclockwise direction.

For example, the first trajectory diagram in FIG. 11(a) where N = 5 is an example.

(7) The nodes of the trajectory include the four vertices of the quadrilateral. Starting from one vertex of the quadrilateral, the trajectory traverses each vertex on the sides of the quadrilateral in one direction. The one direction is a clockwise direction or a counterclockwise direction. For example, the second trajectory diagram in FIG. 11(a) where N = 4 is an example.

(8) The nodes of the trajectory include the three vertices of the triangle. Starting from one vertex of the triangle, the trajectory traverses each vertex of the triangle in one direction. The one direction is a clockwise direction or a counterclockwise direction.

For example, the first trajectory diagram in FIG. 11(a) where N = 3 is an example. For another example, the second trajectory diagram in FIG. 11(a) where N = 3 is also an example.

(9) The nodes of the trajectory include the three vertices of the triangle and one or more nodes located on one or more sides of the triangle. Starting from one node of the triangle, the trajectory traverses the nodes on the triangle in one direction. The one direction is a clockwise direction or a counterclockwise direction.

For example, the third trajectory diagram in FIG. 11(a) where N = 5 is an example. For another example, the third trajectory diagram in FIG. 11(a) where N = 4 is also an example. For yet another example, the fourth trajectory diagram in FIG. 11(a) where N = 4 is also an example.

In some examples, when the preset pattern on the surface of the chip is a quadrilateral-arranged dot matrix, the preset movement rule includes: the trajectory being parallel to one side of the quadrilateral. The four trajectory diagrams in FIG. 11(b) are the four corresponding examples. In some embodiments, when the preset pattern on the surface of the chip is a triangle-arranged dot matrix, the preset movement rule includes: the turning points of the trajectory as a whole forming a hexagon or triangle, or the trajectory being parallel to one side of the triangle.

FIG. 12 illustrates an example in which the preset pattern on the surface of the chip is a triangle-arranged dot matrix. Any two adjacent points of the same row, together with another point of an adjacent row, form a triangular structure. In some embodiments, the other point of the adjacent row is located on the perpendicular bisector of the line connecting the two points in the same row, forming an equilateral triangle.

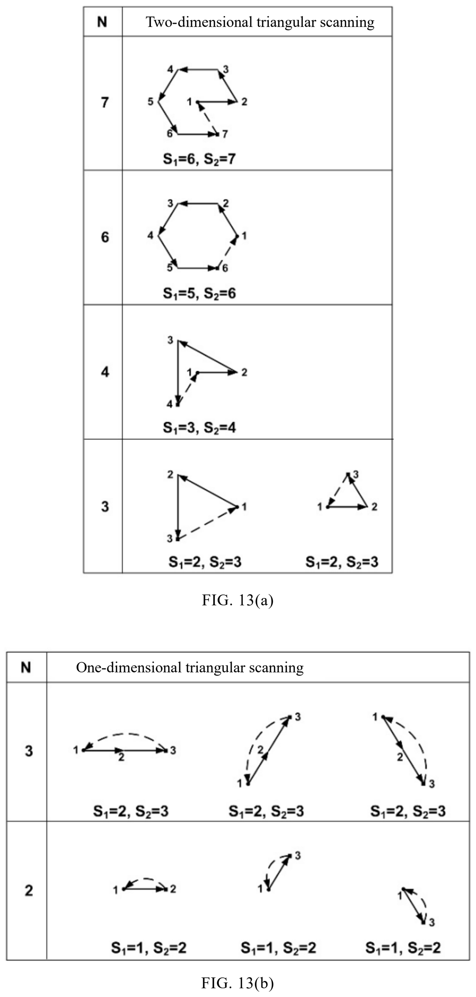

In conjunction with FIGS. 13(a) and 13(b), the trajectory involved in the preset movement rule is explained hereinafter. It should be noted that in the figure, N represents the number of nodes in the trajectory. Understandably, two adjacent nodes can define a one-step path. The sequence number of each node on the trajectories is also marked in the figure, and the movement direction is indicated by arrows on the trajectories.

In some examples, when the preset pattern on the surface of the chip is a triangle-arranged dot matrix, the preset movement rule includes the following: the turning points of the trajectory as a whole form a hexagon or triangle. In some examples, the turning points of the trajectory as a whole forming a hexagon or a triangle includes any of the following:

(1) The nodes of the trajectory include the center and six vertices of the hexagon. Starting from the center of the hexagon, the trajectory reaches one of the vertices and then traverses each vertex of the hexagon in one direction, or vice versa. The one direction is a clockwise direction or a counterclockwise direction.

For example, the trajectory diagram in FIG. 13(a) where N = 7 is an example.

(2) The nodes of the trajectory include all the vertices of the hexagon. Starting from one vertex of the hexagon, the trajectory traverses each vertex of the hexagon in one direction. The one direction is a clockwise direction or a counterclockwise direction.

For example, the trajectory diagram in FIG. 13(a) where N = 6 is an example.

(3) The nodes of the trajectory include the three vertices of the triangle. Starting from one vertex of the triangle, the trajectory traverses each vertex of the triangle in one direction. The one direction is a clockwise direction or a counterclockwise direction.

For example, the two trajectory diagrams in FIG. 13(a) where N = 3 are two corresponding examples.

(4) The nodes of the trajectory include the center and three vertices of the triangle. Starting from the center of the triangle, the trajectory reaches one of the vertices and then traverses each vertex of the triangle in one direction, or vice versa. The one direction is a clockwise direction or a counterclockwise direction.

For example, the trajectory diagram in FIG. 13(a) where N = 4 is an example.

In some examples, when the preset pattern on the surface of the chip is a triangle-arranged dot matrix, the preset movement rule includes: the trajectory being parallel to one side of the triangle. The six trajectory diagrams in FIG. 13(b) are the six corresponding examples.

The above provides some explanations regarding the trajectories involved in the preset movement rules.

When moving along the trajectories involved in the preset movement rules, there are various strategies, such as the strategy of returning to the origin and the strategy of returning along the original path. Details are as follows.

In some embodiments, the preset movement rule includes a first point, a second point, and a trajectory between the first point and the second point. Under each field of view, the preset movement rule is: starting from the first point and moving to the second point along the preset trajectory between the first point and the second point.

That is, for any field of view F of the sample of interest, the preset movement rule corresponding to the field of view F is: starting from the first point and moving to the second point along the preset trajectory between the first point and the second point.

In some embodiments, for any two adjacent fields of view (referred to here as field of view F1 and field of view F2), while acquiring one set of images for one field of view F1, the light modulator 10 is controlled to gradually move from the first point to the second point according to the preset movement rule to acquire one set of images for the field of view F1. After the last raw image of the field of view F1 is acquired, the light modulator 10 is controlled to move back to the first point, and the sample of interest is controlled to move to switch to the next field of view F2. While acquiring one set of images for the next field of view F2, the light modulator 10 is controlled to gradually move from the first point to the second point according to the preset movement rule to acquire one set of images for the next field of view F2. It can be understood that in the embodiments of the present application, the gradual movement of the light modulator 10 is relative to the preset movement rule, which means that moving from one node of the preset movement rule to another node can be regarded as a one-step movement of the light modulator 10.

The above provides some explanation regarding changing the patterned illumination light projected (or cast) onto the field of view F by controlling the movement of the light modulator 10 and switching fields of view by controlling the movement of the stage 61.

Since during the acquisition of a plurality of raw images for each field of view, the light modulator 10 always gradually moves from the first point to the second point (each movement can change or update the patterned illumination light projected onto the field of view), and this also means that when switching from one field of view to the next field of view, the light modulator 10 needs to be moved back from the last node of the trajectory (i.e., the aforementioned second point) to the first node of the trajectory (i.e., the aforementioned first point). Therefore, such a movement strategy is referred to as the strategy of returning to the origin.

In some embodiments, for any two adjacent fields of view (referred to here as field of view F1 and field of view F2), while acquiring one set of images for one field of view F1, the sample of interest is controlled to gradually move from the first point to the second point according to the preset movement rule to acquire one set of images for the field of view F1. After the last raw image of the field of view F1 is acquired, the sample of interest is controlled to move back to the first point, and the sample of interest is controlled to move to switch to the next field of view F2. While acquiring one set of images for the next field of view F2, the sample of interest is controlled to gradually move from the first point to the second point according to the preset movement rule to acquire one set of images for the next field of view F2.

The above provides some explanations regarding the switching of fields of view by, for example, driving the movement of the stage 61 via a driving apparatus 60, as well as the changing of the patterned illumination light projected (or cast) onto the field of view F by controlling the movement of the stage 61.

Since during the acquisition of a plurality of raw images for each field of view, the sample of interest always gradually moves from the first point to the second point (each movement can change or update the patterned illumination light projected onto the field of view), and this also means that when switching from one field of view to the next field of view, the sample of interest needs to be moved back from the last node of the trajectory (i.e., the aforementioned second point) to the first node of the trajectory (i.e., the aforementioned first point). Therefore, such a movement strategy is referred to as the strategy of returning to the origin.

In some embodiments, the preset movement rule includes a first point, a second point, and a trajectory between the first point and the second point. In the case of two adjacent fields of view, the preset movement rule for one field of view is: starting from the first point and moving to the second point along the preset trajectory between the first point and the second point; the preset movement rule for the other field of view is: starting from the second point and moving to the first point along the preset trajectory between the first point and the second point.

That is, for any two adjacent fields of view, one field of view follows the preset trajectory to move from the first point to the second point, while the other field of view follows the preset trajectory to return along the original path, moving from the second point back to the first point. Therefore, such a movement strategy is referred to as the strategy of returning along the original path.

In some embodiments, for any two adjacent fields of view (referred to here as field of view F1 and field of view F2), while acquiring one set of images for one field of view F1, if the preset movement rule for the field of view F1 is to move from the first point to the second point along the preset trajectory between the first point and the second point, then the light modulator 10 is controlled to gradually move from the first point to the second point according to the preset movement rule to acquire one set of images for the field of view F1. After the last raw image of the field of view F1 is acquired, the sample of interest is controlled to move to switch to the next field of view F2. While acquiring one set of images for the next field of view F2, the light modulator 10 is controlled to gradually move from the second point to the first point according to the preset movement rule to acquire the one set of images for the next field of view F2.

In some embodiments, for any two adjacent fields of view (referred to here as field of view F1 and field of view F2), while acquiring one set of images for one field of view F1, if the preset movement rule for the field of view F1 is to move from the first point to the second point along the preset trajectory between the first point and the second point, then the light modulator 10 is controlled to gradually move from the second point to the first point according to the preset movement rule to acquire one set of images for the field of view F1. After the last raw image of the field of view F1 is acquired, the sample of interest is controlled to move to switch to the next field of view F2. While acquiring one set of images for the next field of view F2, the light modulator 10 is controlled to gradually move from the first point to the second point according to the preset movement rule to acquire the one set of images for the next field of view F2.

The above provides some explanation regarding changing the patterned illumination light projected (or cast) onto the field of view F by controlling the movement of the light modulator 10 and switching fields of view by controlling the movement of the stage 61.

In some embodiments, for any two adjacent fields of view (referred to here as field of view F1 and field of view F2), while acquiring one set of images for one field of view F1, if the preset movement rule for the field of view F1 is to move from the first point to the second point along the preset trajectory between the first point and the second point, then the sample of interest is controlled to gradually move from the first point to the second point according to the preset movement rule to acquire one set of images for the field of view F1. After the last raw image of the field of view F1 is acquired, the sample of interest is controlled to move to switch to the next field of view F2. While acquiring one set of images for the next field of view F2, the sample of interest is controlled to gradually move from the second point to the first point according to the preset movement rule to acquire the one set of images for the next field of view F2.

In some embodiments, for any two adjacent fields of view (referred to here as field of view F1 and field of view F2), while acquiring one set of images for one field of view F1, if the preset movement rule for the field of view F1 is to move from the second point to the first point along the preset trajectory between the first point and the second point, then the sample of interest is controlled to gradually move from the second point to the first point according to the preset movement rule to acquire one set of images for the field of view F1. After the last raw image of the field of view F1 is acquired, the sample of interest is controlled to move to switch to the next field of view F2. While acquiring one set of images for the next field of view F2, the sample of interest is controlled to gradually move from the first point to the second point according to the preset movement rule to acquire one set of images for the next field of view F2.

The above provides some explanations regarding the switching of fields of view by, for example, driving the movement of the stage 61 via a driving apparatus 60, as well as the changing of the patterned illumination light projected (or cast) onto the field of view F by controlling the movement of the stage 61.

In conjunction with some of the trajectory diagrams in FIGS. 11(a), 11(b), 13(a), and 13(b), the strategy of returning to the origin and the strategy of returning along the original path will be explained. It should be noted that in the figures, $S_1$ represents the number of steps in the strategy of returning along the original path, $S_2$ represents the number of steps in the strategy of returning to the origin, and the dashed arrows indicate the path for returning from the last node to the first node in the strategy of returning to the origin.

Specifically, taking any one of the trajectory diagrams in FIG. 11(a) where N = 9 as an example, the number of steps in the strategy of returning along the original path is denoted as $S_1$, and the number of steps in the strategy of returning to the origin is denoted as $S_2$. The trajectory for the strategy of returning along the original path, i.e., $S_1$, is $1 \rightarrow 2 \rightarrow 3 \rightarrow 4 \rightarrow 5 \rightarrow 6 \rightarrow 7 \rightarrow 8 \rightarrow 9 \rightarrow 8 \rightarrow 7 \rightarrow 6 \rightarrow 5 \rightarrow 4 \rightarrow 3 \rightarrow 2 \rightarrow 1$, and the number of steps $S_1 = N - 1 = 8$. The trajectory for the strategy of returning to the origin, i.e., $S_2$, is $1 \rightarrow 2 \rightarrow 3 \rightarrow 4 \rightarrow 5 \rightarrow 6 \rightarrow 7 \rightarrow 8 \rightarrow 9 \rightarrow 1 \rightarrow 2 \rightarrow 3 \rightarrow 4 \rightarrow 5 \rightarrow 6 \rightarrow 7 \rightarrow 8 \rightarrow 9$, and the number of steps $S_2 = N = 9$.

From an overall perspective, the strategy of returning along the original path requires relatively fewer scanning or movement steps, which can shorten the total scanning time. Especially when N is smaller, the total time-saving effect becomes more significant.

During the process of movement along the preset trajectory, displacement errors will accumulate continuously. The strategy of returning to the origin can reduce or even eliminate the cumulative movement errors, thereby improving the displacement precision of each scanning step.

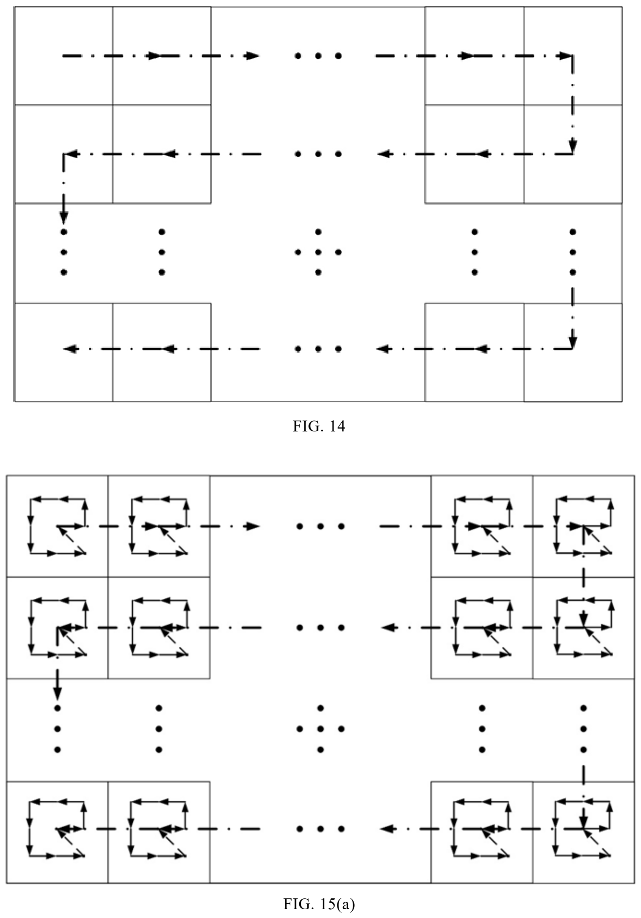

FIG. 14 is a schematic diagram illustrating the switching of fields of view by, for example, driving the movement of the stage 61 via a driving apparatus 60. In the figure, each small square represents one field of view. The stage 61 is driven to move the sample of interest, thereby switching the fields of view one by one until the last field of view.

In some examples where the patterned illumination light projected (or cast) onto the same field of view is changed by controlling the movement of the light modulator 10, and the switching of fields of view is achieved by controlling the movement of the stage 61, the light modulator 10 moves within the same field of view along trajectories such as those shown in FIG. 11(a), 11(b), 13(a), or 13(b). When the switching of fields of view is required, the stage 61 is controlled to move along a path such as that shown in FIG. 14, switching from one field of view to the next field of view.

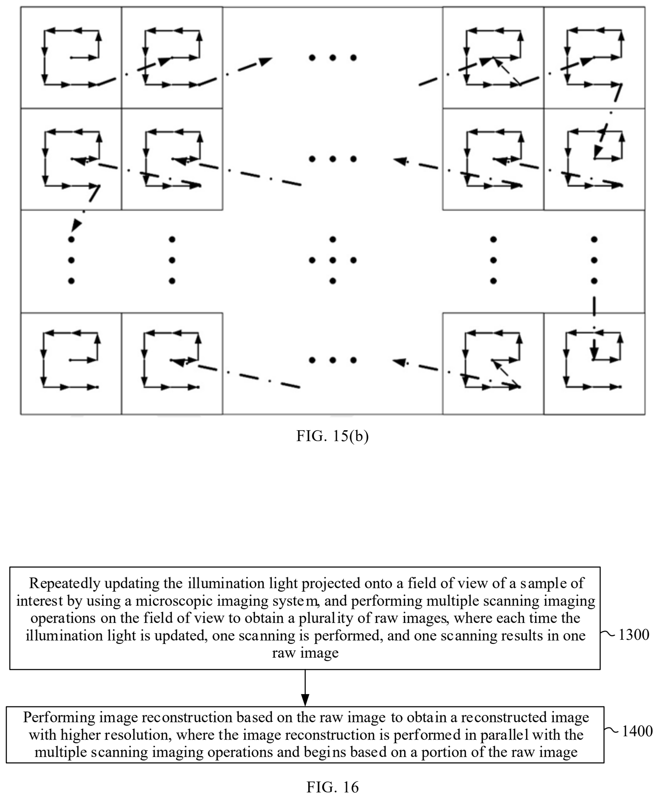

In some examples where the switching of fields of view is performed by, for example, driving the movement of the stage 61 via a driving apparatus 60, and the patterned illumination light projected (or cast) onto the field of view F is changed by controlling the movement of the stage 61, the stage 61 moves within the same field of view along trajectories such as those shown in FIG. 11(a), 11(b), 13(a), or 13(b). When the switching of fields of view is required, the stage 61 is controlled to move along a path such as that shown in FIG. 14, switching from one field of view to the next field of view. FIGS. 15(a) and 15(b) illustrate two examples. FIG. 15(a) illustrates an example where the stage 61 moves within the same field of view along, for example, the first trajectory in FIG. 11(a) where N = 9, and when the switching of fields of view is required, the stage 61 moves along the path shown in FIG. 14, switching from one field of view to the next field of view. After scanning one field of view, the stage 61 will return to the center of the field of view. FIG. 15(b) illustrates an example where the stage 61 moves within the same field of view along, for example, the first trajectory in FIG. 11(a) where N = 9, and the stage 61 moves along the path shown in FIG. 14, switching from one field of view to the next field of view. After scanning one field of view, the stage 61 does not need to return to the center of the field of view but directly switches to the next field of view.

In some embodiments, image acquisition and image reconstruction can be performed in parallel, thereby saving time. It can be understood that the method of image acquisition may include the methods for image acquisition mentioned above or other methods for image acquisition not discussed in the embodiments of the present application. That is, the parallel operation of image acquisition and image reconstruction provided by the embodiments of the present application is not limited by the aforementioned methods for image acquisition.

The parallel operation of image acquisition and image reconstruction is specifically described hereinafter in conjunction with embodiments.

In some embodiments, for two adjacent raw images (referred to here as raw image P1 and raw image P2) in one set of images of any field of view (referred to here as field of view F), the following actions are performed.

Action One: Control the camera 31 to expose and acquire one raw image P1 for the field of view F under the current patterned illumination light.

Action Two: Process the acquired one raw image P1 using a reconstruction algorithm.

Action Three: Control the light modulator 10 to move once to update the patterned illumination light once, so as to prepare for the acquisition of the next raw image P2 for the same field of view F. For example, the light modulator 10 is controlled to move to the next node under a preset movement rule to update the patterned illumination light once, thereby preparing for the acquisition of the next raw image P2 for the same field of view F.

Action Two and Action Three overlap at least partially in the timing and both occur after Action One.

In some embodiments, after performing the actions one to three, the following actions are further performed.

Action Four: Control the camera 31 to expose and acquire the next raw image P2 for the field of view F.

Action Five: Process the acquired next raw image P2 using a reconstruction algorithm.

In some embodiments, in the case where the next raw image P2 is not the last raw image for the field of view F, Action Six is performed. Action Six: Control the light modulator 10 to move once more to update the patterned illumination light once. For example, the light modulator 10 is controlled to move to the next node under a preset movement rule, thereby updating the patterned illumination light once.

Action Five and Action Six overlap at least partially in the timing and both occur after Action Four.

In some embodiments, after performing Action Four, if the next raw image P2 mentioned above is the last raw image for the field of view F, then Action Seven is performed instead of Action Six. Action Seven: Control the sample of interest to move to switch to the next field of view (referred to here as field of view F2), so as to perform parallel image acquisition and reconstruction algorithm for the next field of view F2, until the current field of view is the last field of view.

Action Seven and Action Five overlap at least partially in the timing and both occur after Action Four.

As described above, in some examples, the preset movement rule includes a first point, a second point, and a trajectory between the first point and the second point. Under each field of view, the preset movement rule is: starting from the first point and moving to the second point along the preset trajectory between the first point and the second point. Action Seven further includes: controlling the light modulator 10 to move from the second point back to the first point. Alternatively, in the case of two adjacent fields of view, the preset movement rule for one field of view is: starting from the first point and moving to the second point along the preset trajectory between the first point and the second point; the preset movement rule for the other field of view is: starting from the second point and moving to the first point along the preset trajectory between the first point and the second point. Action Seven further includes: controlling the light modulator 10 to remain stationary at its current position.

The above provides some explanation regarding changing the patterned illumination light projected (or cast) onto the field of view F by controlling the movement of the light modulator 10 and switching fields of view by controlling the movement of the stage 61.

In some embodiments, for two adjacent raw images (referred to here as raw image P1 and raw image P2) in one set of images of any field of view (referred to here as field of view F), the following actions are performed.

Action One: Control the camera 31 to expose and acquire one raw image P1 for the field of view F under the current patterned illumination light.

Action Two: Process the acquired one raw image P1 using a reconstruction algorithm.

Action Three: Control the movement of the sample of interest once to update the patterned illumination light once, so as to prepare for the acquisition of the next raw image P2 for the same field of view F. For example, the stage 61 is controlled to move to the next node under a preset movement rule to update the patterned illumination light once, thereby preparing for the acquisition of the next raw image P2 for the same field of view F.

Action Two and Action Three overlap at least partially in the timing and both occur after Action One.

In some embodiments, after performing the actions one to three, the following actions are further performed.

Action Four: Control the camera 31 to expose and acquire the next raw image P2 for the field of view F.

Action Five: Process the acquired next raw image P2 using a reconstruction algorithm.

In some embodiments, in the case where the next raw image P2 is not the last raw image for the field of view F, Action Six is performed. Action Six: Control the sample of interest to move once more to update the patterned illumination light once. For example, the stage 61 is controlled to move to the next node under a preset movement rule, thereby updating the patterned illumination light once.

Action Five and Action Six overlap at least partially in the timing and both occur after Action Four.

In some embodiments, after performing Action Four, if the next raw image P2 mentioned above is the last raw image for the field of view F, then Action Seven is performed instead of Action Six. Action Seven: Control the sample of interest to move to switch to the next field of view (referred to here as field of view F2), so as to perform parallel image acquisition and reconstruction algorithm for the next field of view F2, until the current field of view is the last field of view.

Action Seven and Action Five overlap at least partially in the timing and both occur after Action Four.

As described above, in some examples, the preset movement rule includes a first point, a second point, and a trajectory between the first point and the second point. Under each field of view, the preset movement rule is: starting from the first point and moving to the second point along the preset trajectory between the first point and the second point. Action Seven further includes: after controlling the sample of interest to move to switch to the next field of view, controlling the sample of interest to move from the second point back to the first point. Alternatively, in the case of two adjacent fields of view, the preset movement rule for one field of view is: starting from the first point and moving to the second point along the preset trajectory between the first point and the second point; the preset movement rule for the other field of view is: starting from the second point and moving to the first point along the preset trajectory between the first point and the second point. Action Seven further includes: after controlling the sample of interest to move to switch to the next field of view, controlling the sample of interest to remain stationary at its current position.

The above provides some explanations regarding the switching of fields of view by, for example, driving the movement of the stage 61 via a driving apparatus 60, as well as the changing of the patterned illumination light projected (or cast) onto the field of view F by controlling the movement of the stage 61.

The above provides some explanations regarding the method of image acquisition according to some embodiments of the present application.

As can be observed, in some examples, image acquisition and image reconstruction can be performed in parallel, thereby saving time.

Therefore, referring to FIG. 16, some embodiments further disclose a method for parallel image acquisition and image reconstruction algorithms, which includes the following steps.

In step 1300, the illumination light projected onto a field of view of a sample of interest is repeatedly updated by using a microscopic imaging system, and multiple scanning imaging operations are performed on the field of view to obtain a plurality of raw images. Each time the illumination light is updated, one scanning is performed, and one scanning results in one raw image.

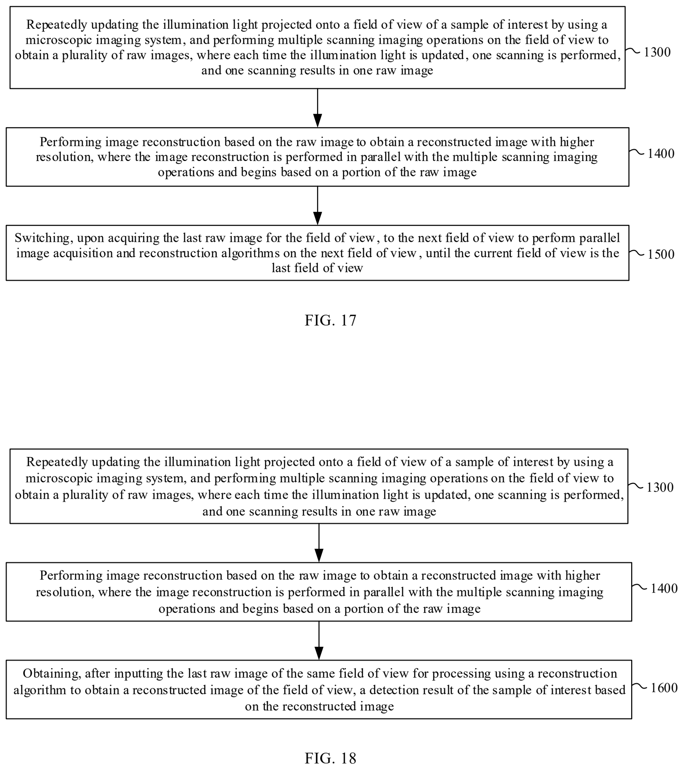

In step 1400, image reconstruction is performed based on the raw image to obtain a reconstructed image with higher resolution. The image reconstruction is performed in parallel with the multiple scanning imaging operations and begins based on a portion of the raw image. Referring to FIG. 17, in some embodiments, the method for parallel image acquisition and image reconstruction algorithms further includes step 1500. In step 1500, upon acquiring the last raw image for a field of view, switch to the next field of view to perform parallel image acquisition and reconstruction algorithms on the next field of view, until the current field of view is the last field of view. In some embodiments, step 1500 is implemented by moving the stage 61 which carries the sample of interest to switch from one field of view to the next field of view.

Referring to FIG. 18, in some embodiments, the method for parallel image acquisition and image reconstruction algorithms further includes step 1600. In step 1600, after processing the last raw image of the same field of view using a reconstruction algorithm to obtain the reconstructed image of the field of view, the detection result of the sample of interest is obtained based on the reconstructed image, such as performing base calling (e.g., based on a base calling algorithm) to obtain base information.

The reconstruction algorithm may be executed herein by a processor, and the processor may be a GPU.

In some embodiments, for any field of view (referred to here as field of view F), step 1400 for parallel image acquisition and reconstruction algorithms can be implemented as follows: Each time a raw image of the field of view F is acquired, the raw image is processed using the reconstruction algorithm, and when the last raw image of the field of view F is acquired, this last raw image is processed using the reconstruction algorithm to obtain the reconstructed image of the field of view F.

In some embodiments, the reconstruction algorithm includes an iterative algorithm.

In some embodiments, for the same field of view (referred to here as field of view F), the reconstruction algorithm iterates at least once for each raw image input of the field of view F.

In some embodiments, for the same field of view (referred to here as field of view F), the first raw image of the field of view F acquired by scanning is used as the initial estimate value of the reconstructed image to perform the iterative algorithm described above.

The reconstruction algorithm will be described in detail hereinafter.

In some examples, the method and process for image acquisition involved in the method for parallel image acquisition and image reconstruction algorithms can refer to some descriptions of the method of image acquisition above, such as those shown in FIG. 9.

For example, for any field of view (referred to here as field of view F), the patterned illumination light that illuminates the sample of interest within the field of view F is updated once, and image acquisition is controlled to obtain one raw image from the current scan of the field of view F. In some embodiments, the patterned illumination light is obtained by passing the illumination light through, for example, the light modulator 10. The light modulator 10 is controlled to move relative to the illumination light or the aperture of the objective lens 24, such that each movement can update the patterned illumination light once. In some embodiments, for any field of view (referred to here as field of view F), multiple scans of the field of view F are completed by controlling the light modulator 10 to move relative to the illumination light or the aperture of the objective lens 24 according to a preset rule (or preset movement rule). The preset movement rule can refer to the descriptions above, which will not be repeated here.

In some embodiments, for two adjacent raw images (referred to here as raw image P1 and raw image P2) of any field of view (referred to here as field of view F), the following actions are performed.

Action one: Control the camera 31 to expose and acquire one raw image P1 for the field of view F under the current patterned illumination light.

Action Two: Process the acquired one raw image P1 using a reconstruction algorithm.

Action Three: Control the light modulator 10 to move once to update the patterned illumination light once, so as to prepare for the acquisition of the next raw image P2 for the same field of view F.

Action Two and Action Three overlap at least partially in the timing and both occur after Action One.

It should be noted that the reconstruction algorithm processor herein may be a GPU. In some embodiments, the reconstruction algorithm processor may be part of the processor 40 herein.

In some embodiments, after performing the actions one to three, the following actions are further performed.

Action Four: Control the camera 31 to expose and acquire the next raw image P2 for the field of view F.

Action Five: Process the acquired next raw image P2 using a reconstruction algorithm.

In some embodiments, in the case where the next raw image P2 is not the last raw image for the field of view F, Action Six is performed. Action Six: Control the light modulator 10 to move once more to update the patterned illumination light once.

Action Five and Action Six overlap at least partially in the timing and both occur after Action Four.

In some embodiments, after performing Action Four, if the next raw image P2 mentioned above is the last raw image for the field of view F, then action seven is performed instead of action six. Action seven: Control the sample of interest to move to switch to the next field of view (referred to here as field of view F2), so as to perform parallel image acquisition and reconstruction algorithm for the next field of view F2, until the current field of view is the last field of view. Action Seven and Action Five overlap at least partially in the timing and both occur after Action Four.

The above actions involve updating the patterned illumination light by controlling the movement of the light modulator 10. This can refer to some descriptions of the method of image acquisition above, such as those shown in FIG. 9, particularly referencing some descriptions regarding the preset movement rule therein.

For example, the preset movement rule includes a first point, a second point, and a trajectory between the first point and the second point. Under each field of view, the preset movement rule is: starting from the first point and moving to the second point along the preset trajectory between the first point and the second point. Action Seven further includes: controlling the light modulator 10 to move from the second point back to the first point. Alternatively, in the case of two adjacent fields of view, the preset movement rule for one field of view is: starting from the first point and moving to the second point along the preset trajectory between the first point and the second point; the preset movement rule for the other field of view is: starting from the second point and moving to the first point along the preset trajectory between the first point and the second point. Action Seven further includes: controlling the light modulator 10 to remain stationary at its current position.

The above explains the parallel image acquisition and image reconstruction from the perspective of the actions performed. The following explains the parallel image acquisition and image reconstruction from the perspective of timing.

In some embodiments, for two adjacent raw images (referred to here as raw image P1 and raw image P2) of any field of view (referred to here as field of view F):

During the first period, control is executed to perform image acquisition to obtain one raw image P1 for the field of view F under the current patterned illumination light.

During the second period, the patterned illumination light is updated once to prepare for the acquisition of the next raw image P2 for the same field of view F.

During the third period, the one raw image P1 acquired during the first period is processed using a reconstruction algorithm.

The second period and the third period at least partially overlap, and both occur after the first period.

In some embodiments:

During the fourth period, control is executed to perform image acquisition to obtain the next raw image P2.

During the fifth period, in the case where the next raw image P2 is not the last raw image for the field of view F, then the patterned illumination light is updated once to prepare for the acquisition of the subsequent raw image for the same field of view (i.e., the raw image following the raw image P2). If the next raw image P2 is the last raw image of the field of view F, then switch to the next field of view (referred to here as field of view F2) to perform parallel image acquisition and reconstruction algorithms for the next field of view F2, until the current field of view is the last field of view.

During the sixth period, the next raw image P2 acquired during the fourth period is processed using a reconstruction algorithm.

The fourth period follows the third period. The fifth period and the sixth period at least partially overlap, and both occur after the fourth period.

The above provides some explanations regarding the method for parallel image acquisition and image reconstruction algorithms.

As can be observed, in terms of hardware, this involves camera exposure and the movement of the light modulator 10 and/or the stage 61 driven via a driving apparatus 60; in terms of algorithms, this involves reconstruction algorithms and even further involves base calling algorithms. Using the aforementioned method for parallel image acquisition and image reconstruction algorithms can shorten the total time required for reconstructing the image for each field of view.

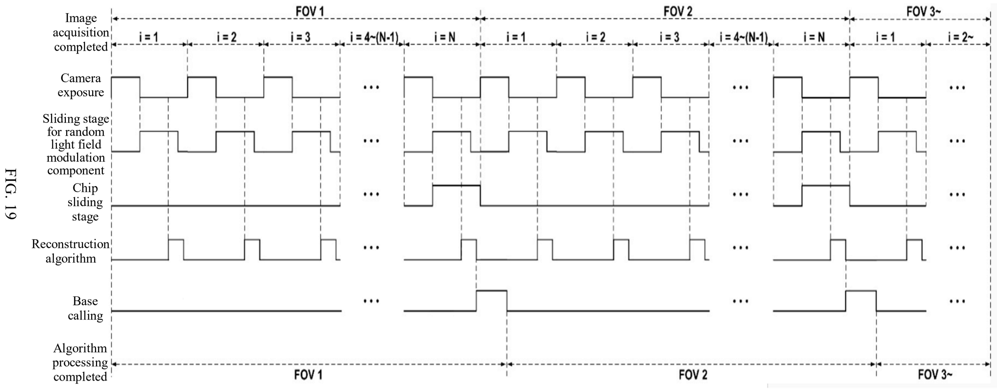

FIG. 19 illustrates an example of a timing diagram for parallel image acquisition and image reconstruction algorithms. In the figure, FOV1, FOV2, and FOV3 represent three adjacent fields of view. The total time required for each field of view is referred to as the "beat time". In the example shown in FIG. 19, the hardware acquires raw images for a plurality of fields of view, and the data for the raw images acquired for each field of view may be set to N. Therefore, within each beat time, the camera needs to expose N times-one camera acquires N raw images, and the N raw images acquired by each camera are used to reconstruct one image. For bases, each base type corresponds to one camera or one reconstructed image. Thus, four cameras, 31a to 31d, acquire 4N raw images, which are used to reconstruct four images. During the acquisition of raw images for each field of view, the patterned illumination light needs to be updated, which involves the movement of the light modulator 10 or the stage 61. In terms of algorithms, four reconstructed images are output through the reconstruction algorithm, that is, each base type corresponds to one reconstructed image. Based on the reconstructed image output from the reconstruction algorithm, the detection result of the sample of interest is obtained. For example, base calling is performed on the reconstructed images using the base calling algorithm to output base sequence information. As can be observed:

(1) After all raw images for the same field of view are acquired, the reconstruction algorithm and the base calling algorithm are locally serial, that is, base calling can be performed on a field of view only after the reconstructed images of the field of view are obtained by the reconstruction algorithm.

(2) The time for "completing algorithm processing" of one field of view overlaps with the time for "completing image acquisition" of the next field of view, but this does not affect the beat time, which is equal to the time for "completing image acquisition".

(3) In some embodiments, for the same field of view, the reconstruction algorithm uses the obtained N raw images as the initial estimate value of the reconstructed image (i.e., post-reconstruction image), which means that the reconstruction algorithm can be performed only after all the N raw images are acquired. In one aspect, the reconstruction algorithm needs to process the N raw images in a centralized manner, which is very time-consuming. In addition, the time for the reconstruction algorithm and the time for the base calling algorithm are serial.

When N is relatively small, the sum of the time for the reconstruction algorithm and the time for the base calling algorithm may exceed the time for image acquisition. In this case, the beat time is prolonged, such that the "beat time" is greater than the time for "completing image acquisition". However, in some embodiments of the present application, for the same field of view (which may be referred to as a field of view F), a first raw image for the field of view F that is acquired by scanning is used as the initial estimate value of the reconstructed image. Therefore, after the first raw image is acquired, the reconstruction algorithm may begin, the subsequent raw images for the field of view F are then acquired step by step, and the reconstruction algorithm is performed step by step. In this way, the original "long time of a whole segment" is divided into "short time of N segments", and the time of the 1st to (N-1)-th segments other than the N-th segment is synchronized with image acquisition, achieving the parallel functionality of hardware and the algorithm, thereby greatly saving time.

Some embodiments of the present application disclose a method for image reconstruction. In some embodiments, the method for image reconstruction can be applied to the sequencing apparatus 100 described in some embodiments herein, but is not limited to sequencing applications, that is, the method for image reconstruction can be used in other application fields. In some embodiments, the image reconstruction mentioned in the foregoing image acquisition and image reconstruction may be performed in parallel. The method for reconstructing an image may be the method for image reconstruction provided in this embodiment.

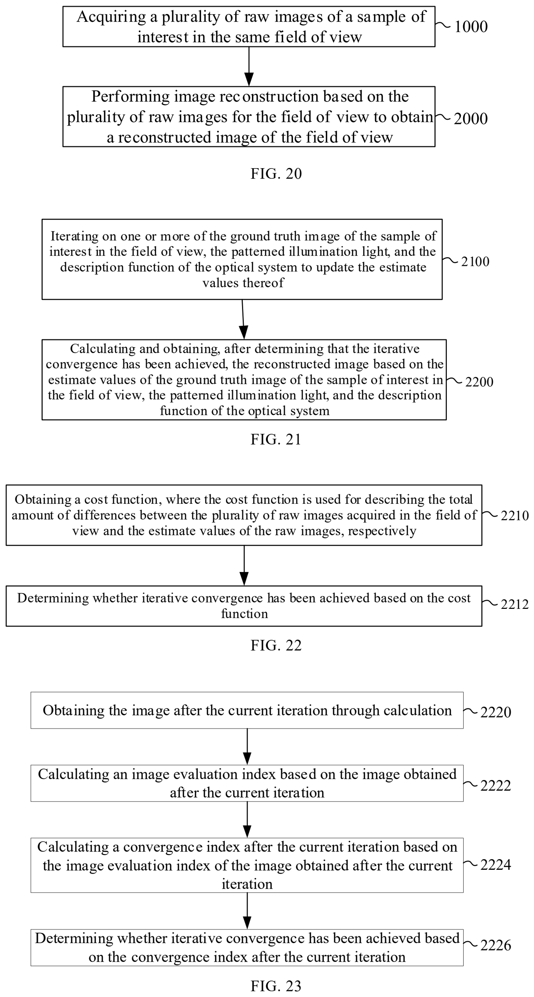

Referring to FIG. 20, the method for image reconstruction in some embodiments includes the following steps.

In step 1000, a plurality of raw images of a sample of interest in the same field of view is acquired.

As described above, the field of view (FOV) is a concept in the optical imaging field, which can be directly understood as the region observable by the image acquisition apparatus, such as a camera, during the optical imaging (photographing) of a target object by an imaging system, that is, the visible region or the scope of vision, i.e., the field of view.

In some implementations, when performing optical imaging on the sample of interest, a single field of view may not cover the entire area of the sample of interest. Therefore, when performing optical imaging on the sample of interest, the sample of interest is divided into a plurality of fields of view for image acquisition. Specifically, the image acquisition apparatus, after acquiring the raw images of one field of view of the sample of interest, switches to the next field of view of the sample of interest and continues acquiring raw images, until raw images of all fields of view of the sample of interest are acquired.

In some embodiments, different regions of the sample of interest can be imaged, for example, by driving the movement of the stage 61 via a driving apparatus 60, thereby enabling the switching of fields of view, that is, switching from one field of view to the next field of view.

In the embodiments of the present application, the sample of interest may be a sample that inherently has a certain solid-phase shape, such as a surface to be tested, or a sample that requires fixation on another carrier, such as a chip, like nucleic acid molecules. The present application does not impose limitations in this regard.

It should be understood that the term "raw image" as referred to in the embodiments of the present application refers to the unprocessed images obtained after imaging by the image acquisition apparatus.

For ease of description, the same field of view (single field of view) mentioned in above step 1000 may be referred to as a field of view F. Understandably, the field of view F may be any field of view of the sample of interest.

In some embodiments, the raw images are acquired in the field of view F after the sample of interest is illuminated with a patterned illumination light.

In some embodiments, the patterned illumination light is obtained by passing an illumination light through a light modulator. The light modulator may be the light modulator 10 disclosed in some embodiments above. For example, the light modulator 10 includes a body 11, and the body 11 includes a plurality of first regions 11a and a plurality of second regions 11b randomly distributed on the body 11. The first region 11a has a first light transmittance, and the second region 11b has a second light transmittance. The first light transmittance is different from the second light transmittance. To save space, the composition, types, and optional configurations of the light modulator 11 will not be repeated here.

By projecting the patterned illumination light onto the sample of interest, a plurality of raw images are acquired in the field of view F. In some embodiments, in the process of acquiring the plurality of raw images for the field of view F, the sample of interest is scanned step by step in the field of view F according to a preset rule, and one of the raw images for the field of view F is obtained for each scan.

In some embodiments, the light modulator 10 is controlled to move step by step relative to the illumination light or the aperture of the objective lens 24, and the scan is completed once each time the light modulator moves. That is, each time the light modulator moves, one raw image for the field of view F is acquired, and then another raw image for the field of view F is acquired after another movement, until the last raw image for the field of view F is acquired.

In some embodiments, the patterned illumination light changes once with each movement. Therefore, the raw images for the field of view F are acquired under different patterned illumination lights.

In some embodiments, in the process of controlling the light modulator 10 to move step by step relative to the illumination light or the aperture of the objective lens 24, the light modulator moves according to a preset movement rule, and the patterned illumination light projected (or cast) onto the field of view F changes once with each movement. This is described in further detail below.

Understandably, by scanning the sample of interest in the field of view F according to the preset rule step by step, after the last raw image for the field of view F is obtained at the last scanning step, the sample of interest is switched from the field of view F to the next field of view until the last field of view. As described above, the switching of fields of view can be achieved, for example, by driving the movement of the stage 61 via a driving apparatus 60, that is, switching from one field of view to the next field of view.

The above provides some explanation regarding changing the patterned illumination light projected (or cast) onto the field of view F by controlling the movement of the light modulator 10 and switching fields of view by controlling the movement of the stage 61.

In some embodiments, the sample of interest is controlled to move step by step relative to the illumination light or the aperture of the objective lens 24, and the scan is completed once each time the sample of interest moves. That is, each time the sample of interest moves, one raw image for the field of view F is acquired, and then another raw image for the field of view F is acquired after another movement, until the last raw image for the field of view F is acquired. In some embodiments, the movement of the sample of interest can be achieved by controlling the movement of the stage 61 carrying the sample of interest.

In some embodiments, the patterned illumination light changes once with each movement. Therefore, the raw images for the field of view F are acquired under different patterned illumination lights.

In some embodiments, in the process of controlling the sample of interest to move step by step relative to the illumination light or the aperture of the objective lens 24, the sample of interest moves according to a preset movement rule, and the patterned illumination light projected (or cast) onto the field of view F changes once with each movement. This is described in further detail below.

Understandably, by scanning the sample of interest in the field of view F according to the preset rule step by step, after the last raw image for the field of view F is obtained at the last scanning step, the sample of interest is switched from the field of view F to the next field of view until the last field of view.

Therefore, switching fields of view can be achieved, for example, by driving the movement of the stage 61 via a driving apparatus 60, i.e., switching from one field of view to the next field of view; in addition, the patterned illumination light projected (or cast) onto the field of view F can be altered by controlling the movement of the stage 61.

In some embodiments, in step 1000, one set of images of the sample of interest in a field of view may be acquired by the method of image acquisition in some embodiments described above, and each set of images includes a plurality of raw images.

It can be seen that step 1000 is used to acquire an image. In some embodiments, step 1000 may be completed by the method of image acquisition as described above.

In some embodiments, step 1000 is used to acquire an image, and the following step 2000 is used to perform image reconstruction. Step 1000 and step 2000 may be performed in serial or in parallel. In the case of being performed in parallel, the steps can be completed through the method for parallel image acquisition and image reconstruction algorithms according to some embodiments as described above.

In step 2000, image reconstruction is performed based on the plurality of raw images for the field of view to obtain a reconstructed image of the field of view.

Therefore, for any field of view, image reconstruction can be performed based on one set of images for the field of view to obtain a post-reconstruction image (i.e., reconstructed image) of the field of view in step 2000.

In some embodiments, the reconstructed image herein is a super-resolution reconstructed image or a super-resolution image.

In step 2000, image reconstruction may be completed through a reconstruction algorithm.

Step 2000 is described in detail below.

Referring to FIG. 21, step 2000 includes the following steps.

In step 2100, one or more of the ground truth pattern of the sample of interest in the field of view F, the patterned illumination light, and the description function of the optical system are iterated to update the estimate values thereof.

The ground truth pattern refers to a pattern displayed in the real physical world by fluorescence signals after the sample of interest is excited under the illumination of the patterned illumination light to generate the fluorescence signals. In the process of photographing the ground truth pattern in the real physical world by the camera, the information of the ground truth pattern is lost, that is, a raw image obtained by photographing the ground truth pattern in the real physical world by the camera cannot completely and truly present the ground truth pattern in the real physical world. Image reconstruction is performed by using a plurality of raw images to improve the resolution of the image, such that the resolution of the reconstructed image is improved as much as possible, and thus the reconstructed image can present the ground truth pattern in the real physical world as nearly lossless as possible.

In some embodiments, in step 2100, one or more of the ground truth pattern of the sample of interest in the field of view F, the patterned illumination light, and the description function are iterated based on a difference function to update the estimate values thereof. For example, one or more of the ground truth pattern of the sample of interest in the field of view F, the patterned illumination light, and the description function are iterated by using a random gradient descent algorithm based on the difference function to update the values of estimation thereof, abbreviated as the estimate values below.

A specific example may be performed as follows:

$$imgSR^{update} = imgSR - k_1 \cdot \frac{\partial C_i}{\partial imgSR}$$

and/or,

$$rpSR^{update} = rpSR - k_2 \cdot \frac{\partial C_i}{\partial rpSR}$$

and/or,

$$PSFSR^{update} = PSFSR - k_3 \cdot \frac{\partial C_i}{\partial PSFSR}$$

where *imgSR, rpSR,* and *PSFSR* represent the estimate value of the ground truth pattern of the sample of interest in the field of view F, the estimate value of the patterned illumination light, and the estimate value of the description function, respectively; *imgSR$^{update}$, rpSR$^{update}$,* and *PSFSR$^{update}$* represent the estimate value of the ground truth pattern of the sample of interest in the field of view F, the estimate value of the patterned illumination light, and the estimate value of the description function after one iteration, respectively; $C_i$ represents the difference between the i-th raw image acquired in the field of view and the estimate value of the raw image; $k_1$, $k_2$, and $k_3$ are iterative steps.

A specific example may also be performed as follows:

$$imgSR^{update} = imgSR - sf_1 \cdot k_1 \cdot \frac{\partial C_i}{\partial imgSR}$$

and/or,

$$rpSR^{update} = rpSR - sf_2 \cdot k_2 \cdot \frac{\partial C_i}{\partial rpSR}$$

and/or,

$$PSFSR^{update} = PSFSR - sf_3 \cdot k_3 \cdot \frac{\partial C_i}{\partial PSFSR}$$

where *imgSR, rpSR,* and *PSFSR* represent the estimate value of the ground truth pattern of the sample of interest in the field of view F, the estimate value of the patterned illumination light, and the estimate value of the description function, respectively; *imgSR$^{update}$, rpSR$^{update}$,* and *PSFSR$^{update}$* represent the updated estimate value of the ground truth pattern of the sample of interest in the field of view F, the updated estimate value of the patterned illumination light, and the updated estimate value of the description function in one iteration process, respectively; $C_i$ represents the difference between the i-th raw image acquired in the field of view and the estimate value of the raw image; $k_1$, $k_2$, and $k_3$ are iterative steps; $sf_1$, $sf_2$, and $sf_3$ are step coefficients of the iterative step $k_1$, the iterative step $k_2$, and the iterative step $k_3$, respectively.

In some embodiments, the iterative step $k_1$ is defined by the initial estimate value of the patterned illumination light. In some embodiments, the iterative step $k_2$ is defined by the initial estimate value of the ground truth pattern of the sample of interest. In some embodiments, the iterative step $k_3$ is defined by the initial estimate value of the raw image. For example:

$$k_1 = \frac{1}{|\max(rpSR)|^2}$$

and/or,

$$k_2 = \frac{1}{|\max(imgSR)|^2}$$

and/or,

$$k_3 = \frac{1}{|max(\mathcal{F}^2(imgSR \cdot rpSR))|^2}$$

where $\mathcal{F}^2$ represents a two-dimensional Fourier transform, and max represents the meaning of taking the maximum value. When processed by a computer or a processor, rpSR, imgSR, and $\mathcal{F}^2$ (*imgSR* ·*rpSR*) are represented in the form of a matrix, and max refers to an element taking the maximum value in the matrix.

In some embodiments, the initial estimate value of the ground truth pattern of the sample of interest in the field of view F is:

$imgSR = \frac{\sum_1^N imgDF_i}{N}$ , where $imgDF_i$ represents the i-th raw image acquired in the field of view F, and N represents the number of the raw images acquired in the field of view F (understandably, this is for the same base); or, $imgSR = imgDF_1$, where $imgDF_1$ represents the first raw image acquired in the field of view F.

In some embodiments, the initial estimate value of the patterned illumination light is:

$$rpSR = 1$$

In some embodiments, the initial estimate value of the description function is:

$$PSFSR = \mathcal{F}^2(imgSR)|_{f_{cutoff} \leq f_{q \cdot \max|\mathcal{F}^2(imgSR)|}}$$

which represents that the initial estimate value *imgSR* of the ground truth pattern of the sample of interest is subjected to a two-dimensional Fourier transform to obtain a spectrum, and the cutoff frequency $f_{cutoff}$ of *PSFSR* is at the spectrum position $f_{q \cdot \max|\mathcal{F}^2(imgSR)|}$ where the amplitude is q times the maximum amplitude of the spectrum, where $\mathcal{F}^2$ represents the two-dimensional Fourier transform, and q is a constant. In some embodiments, $0 < q \leq 10\%$. In some embodiments, q = 5%.

In some embodiments, the description function of the optical system is a point spread function corresponding to the optical transfer function of the optical coefficient. In some embodiments, the optical transfer function of the optical coefficient is generated based on the numerical aperture NA value of the objective lens.

In some embodiments, the step coefficient $sf_1$, the step coefficient $sf_2$, and the step coefficient $sf_3$ are selected from a preset step coefficient set.

In some embodiments, the difference function is used for describing the difference between the acquired raw image and the estimate value of the raw image, and the estimate value of the raw image is calculated based on the estimate value of the ground truth pattern of the sample of interest in the field of view F, the estimate value of the patterned illumination light, and the estimate value of the description function.

In some embodiments, the difference function is $C_i = |imgDF_i - (imgSR \cdot rpSR) * PSFSR|^2$, where $C_i$ represents the difference between the i-th raw image acquired in the field of view F and the estimate value of the raw image, and $imgDF_i$ represents the i-th raw image acquired in the field of view F; $imgSR$, $rpSR$, and $PSFSR$ represent the estimate value of the ground truth pattern of the sample of interest in the field of view F, the estimate value of the patterned illumination light, and the estimate value of the description function, respectively.

It may be understood that in the process of iterating one or more of the ground truth pattern of the sample of interest in the field of view F, the patterned illumination light, and the description function of the optical system to update the estimate values thereof, if the iteration does not achieve convergence, the iteration needs to be performed all the time. In two adjacent iterations, the estimate value of the ground truth pattern of the sample of interest in the field of view F, the estimate value of the patterned illumination light, and the estimate value of the description function obtained in the previous update are used as the input value of the next iteration to continue to update the estimate value of the ground truth pattern of the sample of interest in the field of view F, the estimate value of the patterned illumination light, and the estimate value of the description function.

In step 2200, after determining that iterative convergence is achieved, the reconstructed image is obtained through calculation based on the estimate values of the ground truth pattern of the sample of interest in the field of view F, the patterned illumination light, and the description function of the optical system. That is, after determining that iterative convergence is achieved, the reconstructed image is obtained through calculation based on the latest estimate value of the ground truth pattern of the sample of interest in the field of view F, the latest estimate value of the patterned illumination light, and the latest estimate value of the description function of the optical system.

There may be one or more determination strategies to determine whether iterative convergence is achieved, which are described in detail below.

Referring to FIG. 22, in some embodiments, in step 2200, whether iterative convergence is achieved is determined by the following steps.

In step 2210, a cost function is obtained. The cost function is used for describing the total amount of differences between the plurality of raw images acquired in the field of view F and the estimate values of the raw images, respectively.

In some embodiments, the cost function is: $\sum_1^N C_i = \sum_1^N |imgDF_i - (imgSR \cdot rpSR) * PSFSR|^2$ , where $imgDF_i$ represents the i-th raw image acquired in the field of view F, and $imgSR$, $rpSR$, and $PSFSR$ represent the estimate value of the ground truth pattern of the sample of interest in the field of view F, the estimate value of the patterned illumination light, and the estimate value of the description function, respectively; N represents the number of the raw images acquired in the field of view F.

In step 2212, whether iterative convergence is achieved is determined based on the cost function.

As the number of iterations increases, the cost function is closer to 0. To reduce the calculation amount, the number of iterations may be reduced on the premise of obtaining the basically same reconstruction effect. In this way, the reconstruction efficiency can be improved on the premise of ensuring the reconstruction effect. Therefore, in some embodiments, when the cost function is less than a preset cost threshold, the iteration may be terminated, that is, it is determined that iterative convergence is achieved.

Referring to FIG. 23, in some embodiments, in step 2200, whether iterative convergence is achieved is determined by the following steps. For each iteration:

In step 2220, an image after the current iteration (which refers to a reconstructed image after the current iteration) is obtained through calculation.

In step 2222, an image evaluation index is calculated based on the image after the current iteration.

When the effect of the reconstructed image is evaluated, the image evaluation index may be classified into two categories: a reference type and a non-reference type. The reference evaluation index includes: a structure similarity (SSIM), a peak signal-to-noise ratio (PSNR), a mean square error (MSE), and the like. For the reference evaluation index, the evaluation index is obtained by calculating the difference between a reconstructed image and a reference image based on the reference image, and a raw image may be selected as the reference image. The non-reference evaluation index usually includes: a coefficient of variation (CV), image entropy, and the like. For the non-reference evaluation index, the evaluation index is obtained by directly performing feature extraction and calculation on a reconstructed image.

In some embodiments, the image evaluation index mentioned in step 2222 includes a non-reference image evaluation index, such as a coefficient of variation and/or image entropy. In step 2224, based on the image evaluation index of the image after the current iteration, a convergence index after the current iteration is calculated.

In image reconstruction, different image evaluation indexes are greatly different, which is not conducive to visually observing the convergence condition and determining the minimum number of iterations according to the convergence condition. Therefore, in step 2224, the convergence index is further calculated by the image evaluation index.

In some embodiments, in step 2224, the difference between the image evaluation index of the image after the current iteration and the image evaluation index of the image after the previous iteration is calculated, which is referred to as a first difference; in step 2224, the difference between the image evaluation index of the image after the current iteration and the image evaluation index of the image after the first iteration is calculated, which is referred to as a second difference; in step 2224, the difference between the first difference and the second difference is calculated and used as the convergence index.

In step 2226, whether iterative convergence is achieved is determined based on the convergence index after the current iteration.

For example, in step 2224, the percentage of the first difference and the second difference is calculated as the convergence index. In step 2226, when the percentage is greater than a preset percentage, it is determined that iterative convergence is achieved.

An example may be:

Taking the coefficient of variation CV as an example, the convergence factor cf is defined as:

$$cf = \left| \frac{CV_j - CV_{j-1}}{CV_j - CV_1} \right| \times 100\%$$

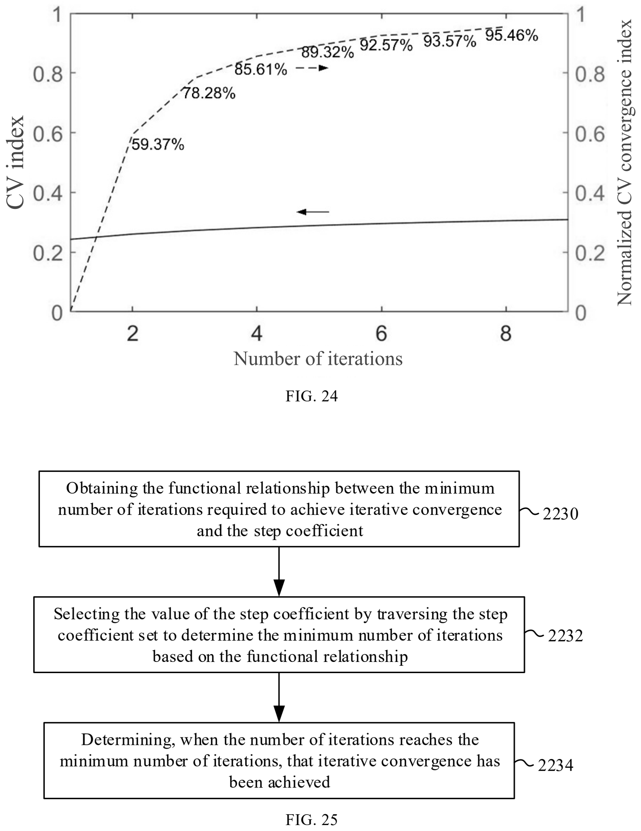

That is, this is to calculate the percentage of $|CV_j - CV_{j-1}|$ (the difference between the CV values of the reconstructed images obtained in the j-th iteration and the j - 1-th iteration) and $|CV_j - CV_1|$ (the difference between the CV values of the reconstructed images obtained in the j-th iteration and the 1st iteration), from which the convergence condition of the reconstruction algorithm is visually shown. In addition, the minimum number of iterations may be quickly determined based on the convergence requirement and the convergence condition. Referring to FIG. 24, to reduce the number of iterations so as to shorten the time consumption of image reconstruction, if cf $\geq$ 90% is required, it is considered that the image reconstruction is completed, and the number of iterations may be reduced to 6; if cf $\geq$ 80% is required, it is considered that the image reconstruction is completed, and the number of iterations may be reduced to 4. It should be noted that in FIG. 24, the left axis refers to the CV value of the reconstructed image, and the curve indicated by a black solid-line arrow represents the curve of the CV value and the number of iterations; the right axis refers to the normalized convergence factor, and the curve indicated by a black dashed-line arrow represents the curve of the convergence factor and the number of iterations.

Referring to FIG. 25, in some embodiments, in step 2200, whether iterative convergence is achieved is determined by the following steps.

In step 2230, the functional relationship between the minimum number of iterations required to achieve iterative convergence and a step coefficient is obtained, where the step coefficient includes one or more of the step coefficient $sf_1$, the step coefficient $sf_2$, and the step coefficient $sf_3$.

In step 2232, the value of the step coefficient is selected by traversing the step coefficient set to determine the minimum number of iterations based on the functional relationship.

In some embodiments, the functional relationship is established based on the step coefficient, the number of iterations, and the image evaluation index of an image after iteration (i.e., reconstructed image after iteration).

Reference is made to the above description for the image evaluation index, which will not be repeated herein.

In step 2234, when the number of iterations reaches the above minimum number of iterations, it is determined that iterative convergence is achieved.

The random gradient descent method is a nonlinear convergence method. Based on the nonlinear convergence method, a strategy for further accelerating convergence is proposed, which may be referred to as a secondary nonlinear convergence method. Specifically, the iterative steps $k_1$, $k_2$, and $k_3$ are separately multiplied by a step coefficient, where $sf_1$, $sf_2$, and $sf_3$ are the step coefficients of the iterative step $k_1$, the iterative step $k_2$, and the iterative step $k_3$, respectively. By traversing a plurality of step coefficients, the number of iterations is counted under the requirement of a specific convergence factor, and the step coefficient with the minimum number of iterations is the optimal step coefficient, which can achieve faster nonlinear convergence based on the original random gradient descent. The minimum number of iterations *lis* (least iterative steps) required for convergence is the function of the step coefficients $sf_1$, $sf_2$, and $sf_3$ (i.e., the functional relationship mentioned in step 2230):

$$lis = \mathcal{F}(sf_1, \ sf_2, \ sf_3)$$

The minimum number of iterations lis required for convergence is obtained in the three-dimensional space by traversing the step coefficients $sf_1$, $sf_2$, and $sf_3$; the above iterative formula is used to iteratively update the three estimates *imgSR, rpSR, and PSFSR* to achieve fast convergence.

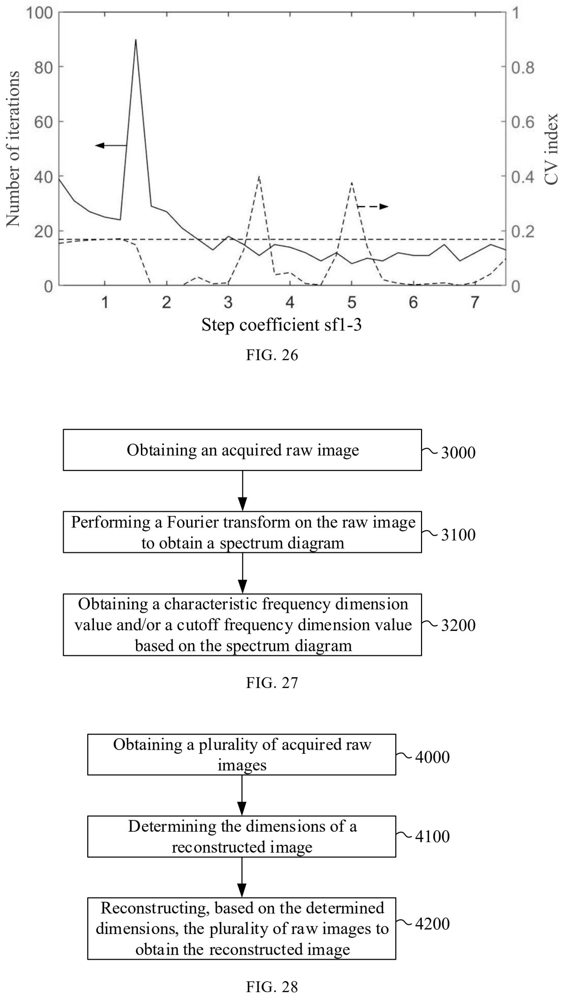

Since the three-dimensional space is not convenient to visually show how to select the optimal step coefficient to achieve the minimum number of iterations, to simplify the test situation, it may constrain $sf_1 = sf_2 = sf_3$. In this case, the number of iterations and the step coefficient are in a single-factor functional relationship, which can be visually shown by a two-dimensional curve graph. In FIG. 26, the step coefficient $sf_1 = sf_2 = sf_3$ traverses the set {0.25, 0.50, 0.75, 1.00, 1.25, 1.50, 1.75, 2.00, 2.25, 2.5, 2.75, 3.00, 3.25, 3.5, 3.75, 4.00, 4.25, 4.5, 4.75, 5.00, 5.25, 5.50, 5.75, 6.00, 6.25, 6.50, 6.75, 7.00, 7.25, 7.5} to count the minimum number of iterations lis for different step coefficients with the convergence factor 0.99 used as a convergence criterion and the CV used as an evaluation index (which is not limited to the CV, and other evaluation indexes may also be used). It should be noted that in FIG. 26, the left axis represents the number of iterations, and the curve indicated by a black solid-line arrow is the curve representing the number of iterations and the step coefficient; the right axis represents the CV indicator, and the curve indicated by a black dashed-line arrow represents the curve of the CV indicator and the step coefficient. It can be seen from the analysis of FIG. 26 that as the step coefficient changes, both the number of iterations and the CV index fluctuate to different degrees. The horizontal dashed-line shows that when the step coefficient is 1, the number of iterations is 25, and the CV index is 0.3378. When the minimum number of iterations is selected, the following needs to be met: (1) The CV index is as large as possible. (2) It is superior to the CV value when the iteration coefficient is 1. (3) The number of iterations is as small as possible. Based on the above principle, when the step coefficient is 3.50, the number of iterations is 11, and the CV index is 0.4002; when the step coefficient is 5.00, the number of iterations is 8, and the CV index is 0.3766. In summary, the step coefficients of 3.50 and 5.00 are both preferred. Considering that the CV indexes are not greatly different as shown by ( $\frac{0.4002-0.3766}{0.4002} = 5.90\%$ ), but the reduction in the number of iterations is relatively large as shown by ( $\frac{11-8}{11} = 27.27\%$ ), the optimal step coefficient should be 5.00. When the step coefficient $sf_1 = sf_2 = sf_3$ is unconstrained, the step coefficients $sf_1$, $sf_2$, and $sf_3$ separately traverse the set {0.25, 0.50, 0.75, 1.00, 1.25, 1.50, 1.75, 2.00, 2.25, 2.5, 2.75, 3.00, 3.25, 3.5, 3.75, 4.00, 4.25, 4.5, 4.75, 5.00, 5.25, 5.50, 5.75, 6.00, 6.25, 6.50, 6.75, 7.00, 7.25, 7.5}, and the same method is used to determine the optimal step coefficient and the minimum number of iterations.

How to determine whether iterative convergence is achieved in step 2200 is described above. For the method for image reconstruction in some embodiments herein, the dimensions of the reconstructed image may be determined by the method for determining dimensions of a reconstructed image in some embodiments herein.

How to determine better dimensions of the reconstructed image is described below.

When the illumination light is projected through the objective lens 24, the super-resolution multiple of the reconstructed image is twice theoretically. Therefore, when image reconstruction is performed, the dimension of the reconstructed image is generally twice the dimension of the raw image, that is, each of the X/Y dimensions is enlarged to twice the original. However, in practical imaging, in one aspect, the numerical aperture (NA) of the objective lens 24 represents the theoretical limit of resolution, and an actual optical system cannot reach the theoretical limit; in another aspect, to clearly image the sample of interest, the minimum distance between adjacent points on the pattern of the sample of interest on the surface of the chip is generally less than the Abbe diffraction limit resolution $R_{Abbe} = \frac{\lambda_{Em}}{2 \cdot NA}$ ($\lambda_{Em}$ is the fluorescence wavelength), and thus the reconstructed image does not need to be enlarged to twice the original.

Therefore, to reduce the data amount and computational load of the reconstructed image, save computing power resources, and improve the reconstruction speed of the reconstruction algorithm, a method for determining dimensions of a reconstructed image (i.e., post-reconstruction image) is provided in some embodiments.

Referring to FIG. 27, in some embodiments, the method for determining dimensions of a reconstructed image includes the following steps.

In step 3000, an acquired raw image is obtained, where the reconstructed image (i.e., post-reconstruction image) is obtained by reconstructing a plurality of raw images.

In step 3100, a Fourier transform is performed on the raw images to obtain a spectrum diagram. In step 3200, a characteristic frequency dimension value $f_{pattern}$ and/or a cutoff frequency dimension value $f_{cutoff}$ is obtained based on the spectrum diagram.

The characteristic frequency dimension value $f_{pattern}$ is the distance between the pixel of the characteristic frequency peak on the spectrum diagram and the center pixel of the spectrum diagram. In some embodiments, the distance may be represented by the number of pixels.

The cutoff frequency dimension value $f_{cutoff}$ is the distance between the pixel of the cutoff frequency on the spectrum diagram and the center pixel of the spectrum diagram. In some embodiments, the distance may be represented by the number of pixels.

In step 3300, the dimensions of the reconstructed image are determined based on the characteristic frequency dimension value $f_{pattern}$ and/or the cutoff frequency dimension value $f_{cutoff}$.

In some examples, in step 3300, one or more frequency dimension ranges are determined based on the characteristic frequency dimension value $f_{pattern}$ and/or the cutoff frequency dimension value $f_{cutoff}$; the one or more frequency dimension ranges include one or more of $2 * f_{pattern}$, $2 * f_{cutoff}$, and ($f_{pattern} + f_{cutoff}$). In step 3300, the dimensions of the reconstructed image are determined based on the one or more frequency dimension ranges.

For example, the reconstructed image is quadrilateral, and the length of one side of the reconstructed image is $2 * f_{pattern}$ pixels, $2 * f_{cutoff}$ pixels, or ($f_{pattern} + f_{cutoff}$) pixels. The length of an adjacent side of the reconstructed image is $2 * f_{pattern}$ pixels, $2 * f_{cutoff}$ pixels, or ($f_{pattern} + f_{cutoff}$) pixels.

For another example, the reconstructed image is circular, and the diameter of the reconstructed image is $2 * f_{pattern}$ pixels, $2 * f_{cutoff}$ pixels, or ($f_{pattern} + f_{cutoff}$) pixels.

In some examples, in step 3300, the dimensions of the reconstructed image are determined based on the constraint of the Fourier transform of a processor for reconstructing the raw image, and the characteristic frequency dimension value $f_{pattern}$ and/or the cutoff frequency dimension value $f_{cutoff}$.

In some embodiments, the processor for reconstructing the raw image and a processor capable of executing the reconstruction algorithm mentioned above may be the same processor.

In some embodiments, the processor for reconstructing the raw image may be a GPU.

In some embodiments, the processor for reconstructing the raw image may form a part of the processor 40 herein.

In some embodiments, in step 3300, the dimensions of the reconstructed image are determined based on the minimum value greater than a first value among values satisfying the constraint of the Fourier transform; the first value is $2 * f_{pattern}$ pixels, $2 * f_{cutoff}$ pixels, or ($f_{pattern} + f_{cutoff}$) pixels.

For example, the reconstructed image is quadrilateral, and the lengths of both sides of the reconstructed image are both the minimum value greater than the first value among the values satisfying the constraint of the Fourier transform.

For another example, the reconstructed image is circular, and the diameter of the reconstructed image is: the minimum value greater than the first value among the values satisfying the constraint of the Fourier transform.

In some embodiments, in step 3300, the dimensions of the reconstructed image are determined based on a value closest to a first value among values satisfying the constraint of the Fourier transform; the first value is $2 * f_{pattern}$ pixels, $2 * f_{cutoff}$ pixels, or $(f_{pattern} + f_{cutoff})$ pixels. This solution can improve processing efficiency on the premise of ensuring precision requirements.

For example, the reconstructed image is quadrilateral, and the lengths of both sides of the reconstructed image are both the value closest to the first value among the values satisfying the constraint of the Fourier transform.

For another example, the reconstructed image is circular, and the diameter of the reconstructed image is: the value closest to the first value among the values satisfying the constraint of the Fourier transform.

In some embodiments, the values satisfying the constraint of the Fourier transform are $2^a * 3^b$, where $a$ and $b$ are both positive integers.

In some embodiments, the reconstructed image is rectangular. In some embodiments, the reconstructed image is square.

Referring to FIG. 28, in some embodiments, a method for image reconstruction is disclosed, including the following steps.

In step 4000, a plurality of acquired raw images are obtained.

For the acquisition of the raw images mentioned in step 4000, the raw images may be acquired by the method of image acquisition or the method for parallel image acquisition and image reconstruction algorithms disclosed in some embodiments herein.

In step 4100, the dimensions of a reconstructed image are determined.

In step 4200, the plurality of raw images are reconstructed based on the dimensions determined in step 4100 to obtain the reconstructed image.

In step 4100, the dimensions of the reconstructed image may be determined by the method for determining dimensions of a reconstructed image in some embodiments described herein.

In some embodiments, the dimensions of the reconstructed image are at least related to a characteristic frequency dimension value $f_{pattern}$ and/or a cutoff frequency dimension value $f_{cutoff}$ associated with the pattern of the sample of interest.

The characteristic frequency dimension value $f_{pattern}$ is the distance between the pixel of the characteristic frequency peak on the spectrum diagram and the center pixel of the spectrum diagram. In some embodiments, the distance may be represented by the number of pixels.

The cutoff frequency dimension value $f_{cutoff}$ is the distance between the pixel of the cutoff frequency on the spectrum diagram and the center pixel of the spectrum diagram. In some embodiments, the distance may be represented by the number of pixels.

In some embodiments, the dimensions of the reconstructed image are related to one or more frequency dimension ranges; the one or more frequency dimension ranges include one or more of $2 * f_{pattern}$, $2 * f_{cutoff}$, and $(f_{pattern} + f_{cutoff})$.

For example, the reconstructed image is quadrilateral, and the length of one side of the reconstructed image is $2 * f_{pattern}$ pixels, $2 * f_{cutoff}$ pixels, or $(f_{pattern} + f_{cutoff})$ pixels. The length of an adjacent side of the reconstructed image is $2 * f_{pattern}$ pixels, $2 * fcutoff$ pixels, or $(f_{pattern} + fcutoff)$ pixels.

For another example, the reconstructed image is circular, and the diameter of the reconstructed image is $2 * f_{pattern}$ pixels, $2 * f_{cutoff}$ pixels, or $(f_{pattern} + f_{cutoff})$ pixels.

In some embodiments, the dimensions of the reconstructed image are further related to the constraint of the Fourier transform of a processor for reconstructing the above reconstructed image (namely, the post-reconstruction image). In some embodiments, the processor for reconstructing the raw image and a processor for the reconstruction algorithm mentioned above may be the same processor. In some embodiments, the processor for reconstructing the raw image may be a GPU. In some embodiments, the processor for reconstructing the raw image may form a part of the processor 40 herein.

In some embodiments, the dimensions of the reconstructed image are determined by the minimum value greater than a first value among values satisfying the constraint of the Fourier transform; the first value is $2 * f_{pattern}$ pixels, $2 * f_{cutoff}$ pixels, or $(f_{pattern} + fcutoff)$ pixels.

For example, the reconstructed image is quadrilateral, and the lengths of both sides of the reconstructed image are both the minimum value greater than the first value among the values satisfying the constraint of the Fourier transform.

For another example, the reconstructed image is circular, and the diameter of the reconstructed image is: the minimum value greater than the first value among the values satisfying the constraint of the Fourier transform.

In some embodiments, the dimensions of the reconstructed image are determined by a value closest to a first value among values satisfying the constraint of the Fourier transform; the first value is 2 * $f_{pattern}$ pixels, 2 * $f_{cutoff}$ pixels, or ($f_{pattern}$ + $f_{cutoff}$) pixels.

For example, the reconstructed image is quadrilateral, and the lengths of both sides of the reconstructed image are both the value closest to the first value among the values satisfying the constraint of the Fourier transform.

For another example, the reconstructed image is circular, and the diameter of the reconstructed image is: the value closest to the first value among the values satisfying the constraint of the Fourier transform.

In some embodiments, the values satisfying the constraint of the Fourier transform are $2^a * 3^b$, where $a$ and $b$ are both positive integers.

In some embodiments, the reconstructed image is rectangular. In some embodiments, the reconstructed image is square.

The following description is provided by taking the acquired raw image having $1024 \times 1024$ pixels and the pattern of the sample of interest on the surface of the chip being arranged in a triangle as an example.

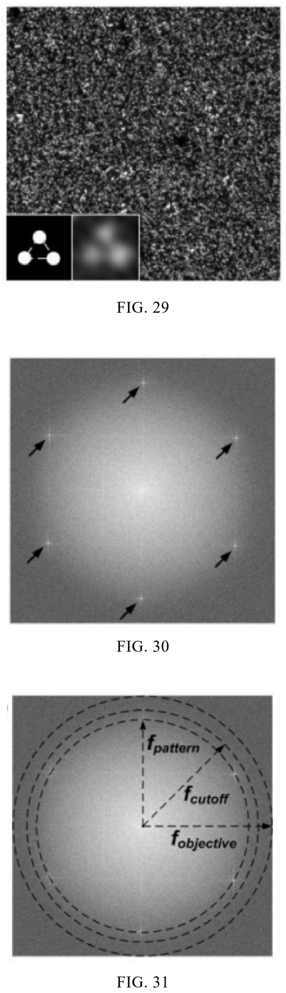

FIG. 29 shows an example of the raw image, the spectrum diagram of which is obtained after a two-dimensional Fourier transform, as shown in FIG. 30. A triangle pattern generates characteristic frequency peaks. As indicated by arrows in FIG. 30, six characteristic frequency peaks are shown in the figure, and characteristic frequency dimension values corresponding to the characteristic frequency peaks are denoted as $f_{pattern}$, which refer to distances between pixels of the characteristic frequency peaks on the spectrum diagram and the center pixels of the spectrum diagram. The cutoff frequency dimension value $f_{cutoff}$ is the distance between the pixel of the cutoff frequency on the spectrum diagram and the center pixel of the spectrum diagram. The diffraction limit frequency dimension value of the objective lens 24 is $f_{objective}$, where $f_{cutoff}$ is greater than and infinitely approximate to $f_{pattern}$. FIG. 31 shows three frequency dimension values, i.e., the characteristic frequency dimension value $f_{pattern}$, the cutoff frequency dimension value $f_{cutoff}$, and the diffraction limit frequency dimension value $f_{objective}$.

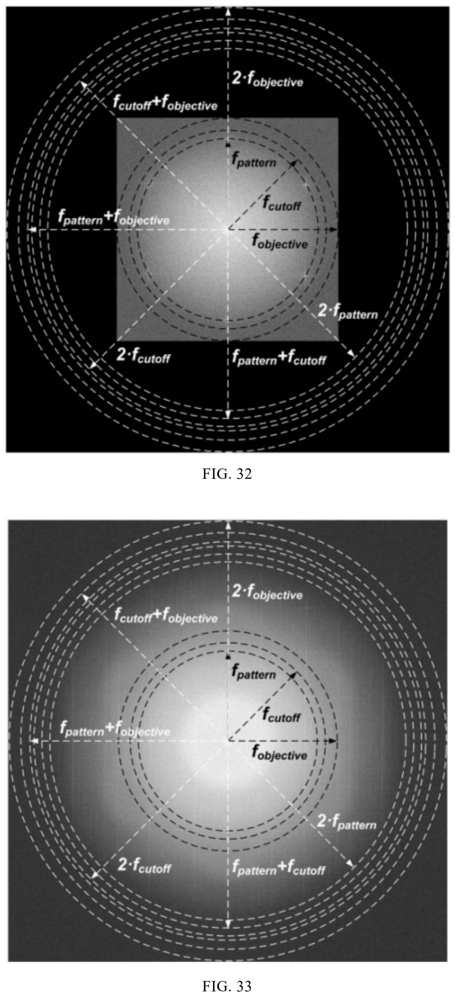

From FIG. 31, the characteristic frequency dimension value $f_{pattern}$, the cutoff frequency dimension value $f_{cutoff}$, and the diffraction limit frequency dimension value $f_{objective}$ can be obtained as 416 pixels, 432 pixels, and 512 pixels, respectively. When the dimensions of the reconstructed image are constructed, the area exceeding the diffraction limit frequency dimension value $f_{objective}$ is assigned a zero value, as shown in FIG. 32; moreover, the frequency dimension ranges of six arrangement combinations are calculated as: $2 f_{pattern} = 832$ , $f_{pattern} + f_{cutoff} = 848$ , $2f_{cutoff} = 864$ , $f_{pattern} + f_{objective} = 928$, $f_{cutoff} + f_{objective} = 924$, and $2 \cdot f_{objective} = 1024$.

To ensure that the dimensions of the reconstructed image are as small as possible and all reconstructed spectrum information is included, $2 \cdot f_{objective}$ is used as an example herein for image reconstruction. It can be seen from FIG. 33 that $f_{pattern} + f_{cutoff}$ can not only include all the reconstructed spectrum information, but also reserve a certain spectrum margin ($f_{cutoff} - f_{pattern}$). In short, the dimensions of the reconstructed image are $1696 \times 1696$ pixels, and do not need to be expanded to $2048 \times 2048$. In this way, the dimensions of the reconstructed image are reduced by 1.46 times.

In addition, in actual engineering practice, to increase the speed of the Fourier transform, a graphic processing unit (graphic processing unit, GPU) may be selected to perform image reconstruction (that is, execute the image reconstruction algorithm), and the Fourier transform is implemented on the graphic processing unit, instead of selecting a central processing unit (CPU).

When image reconstruction is performed on the GPU, to achieve optimal efficiency, the length of the Fourier transform is constrained to be $L = 2^a * 3^b$, where L is the length of the Fourier transform, and is obtained by traversing $a$ and $b$. $a$ and b are both positive integers. Therefore, the dimensions of the reconstructed image need to satisfy $f_{pattern} + f_{cutoff} \leq$ L. In this case, L needs to be as small as possible. Taking the above raw image of $1024 \times 1024$ pixels as an example, the length of the Fourier transform is $L = 2^6 \cdot 3^3 = 1728 \geq f_{pattern} + f_{cutoff}$. Therefore, the optimal dimensions of the reconstructed image are $1728 \times 1728$ pixels. In this case, coincidentally, the dimension of the reconstructed image is the same as that of the reconstructed image obtained by selecting $2 \cdot f_{cutoff}$.

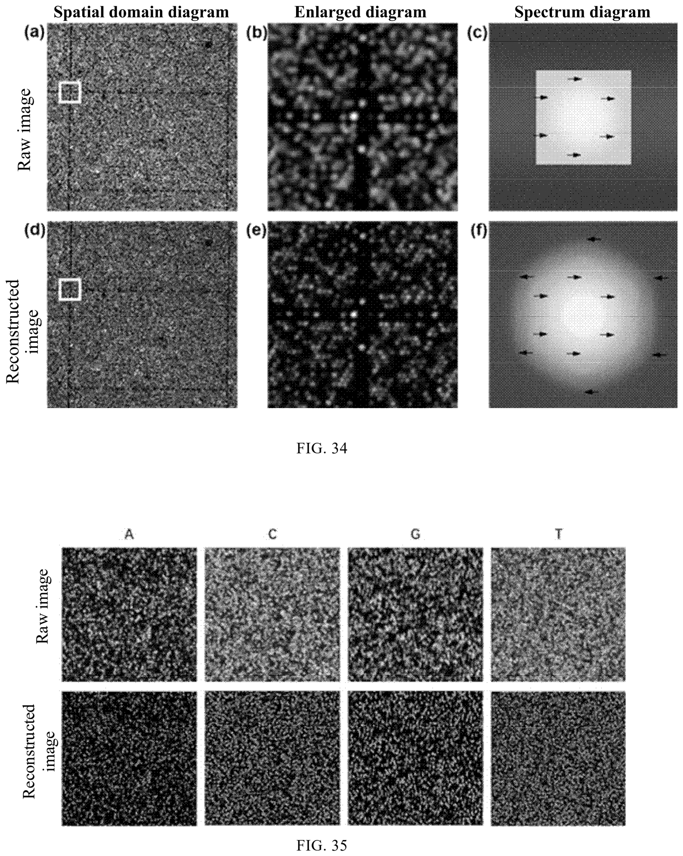

FIG. 34 shows a comparison effect between raw images and reconstructed images in an example.

In FIG. 34, FIG. (a) represents a raw image, FIG. (b) represents a partial enlarged diagram of a region outlined by a white solid line in the raw image, and FIG. (c) represents a two-dimensional spectrum diagram obtained by performing a two-dimensional Fourier transform on the raw image. Since the pattern of the sample of interest on the surface of the chip is arranged in a triangle, six characteristic spectrum peaks (as shown by the black horizontal rightward arrows) are generated on the spectrum diagram, and the characteristic spectrum peaks are abbreviated as spectrum peaks below.

In FIG. 34, FIG. (d) represents a reconstructed image, FIG. (e) represents a partial enlarged diagram of a region outlined by a white solid line in the reconstructed image, and FIG. (f) represents a two-dimensional spectrum diagram obtained by performing a two-dimensional Fourier transform on the reconstructed image. It can be seen that twelve spectrum peaks (as shown by the black horizontal rightward and leftward arrows) are generated on the spectrum diagram of the reconstructed image.

The relationship between the resolution (R) and the frequency (f) is as follows:

$$R = \frac{2 \cdot ps}{f}$$

where ps is the object-side pixel size, and f is the normalized frequency. As can be seen from FIG. (f), there are twelve spectrum peaks in the spectrum, which are distributed in inner and outer rings. The six spectrum peaks in the inner ring are the same as those in FIG. (c) (as shown by the black horizontal rightward arrows), and frequencies of the six spectrum peaks in the outer ring (as shown by the black horizontal leftward arrows) are twice of corresponding frequencies of the six spectrum peaks in the inner ring; the corresponding resolution is also improved by twice. A part of the raw image and a part of the reconstructed image are separately selected for enlargement, as shown in FIG. (b) and FIG. (e). It can be more visually seen from FIG. (b) and FIG. (e) that the resolution of the reconstructed image is improved, thereby achieving a super-resolution effect.

FIG. 35 shows a comparison effect between raw images and reconstructed images in an example. The two images in the first column are the raw image and the reconstructed image for base A, the two images in the second column are the raw image and the reconstructed image for base C, the two images in the third column are the raw image and the reconstructed image for base G, and the two images in the fourth column are the raw image and the reconstructed image for base T. It can be seen from the figure that for the raw images and the reconstructed images of the four base channels of ACGT, the resolution of the reconstructed images has significantly improved, and adjacent unresolved points in the raw images can be well resolved in the reconstructed images; meanwhile, the contrast of the reconstructed images is also significantly higher.

The present disclosure is illustrated with reference to various exemplary embodiments. However, those skilled in the art will recognize that changes and amendments may be made to the exemplary embodiments without departing from the scope of the present disclosure. For example, various operation steps and components for performing the operation steps may be implemented in different ways depending on the particular application or in view of any number of cost functions associated with the operation of the system (e.g., one or more steps may be deleted, modified, or combined into other steps).

The above embodiments can be fully or partially implemented through software, hardware, firmware, or any combination thereof. In addition, as understood by those skilled in the art, the principles herein may be reflected in a computer program product on a computer-readable storage medium. The readable storage medium is pre-loaded with a computer-readable program code. Any tangible, non-transitory computer-readable storage media may be used, including magnetic storage devices (hard disks, floppy disks, etc.), optical storage devices (CDs to ROMs, DVDs, Blu-ray disks, etc.), flash memory, and/or the like. These computer program instructions may be loaded onto a general-purpose computer, a special-purpose computer, or other programmable data processing devices to form a machine, such that these instructions executed on the computer or other programmable data processing apparatuses can generate an apparatus for implementing the specified functions. These computer program instructions may also be stored in a computer-readable memory that can instruct a computer or other programmable data processing devices to operate in a particular manner. In this way, the instructions stored in the computer-readable memory can form a manufactured product including an implementation apparatus for implementing the specified functions. The computer program instructions may also be loaded onto a computer or other programmable data processing devices to perform a series of operation steps on the computer or other programmable devices to generate a process implemented by the computer, such that the instructions executed on the computer or other programmable devices may provide steps for implementing the specified functions.

While the principles herein have been illustrated in various embodiments, many modifications to the structure, arrangement, ratios, elements, materials, and components, particularly adapted to specific environments and operational requirements, can be used without departing from the principles and scope of the present disclosure. The above modifications and other changes or amendments are intended to be included within the scope of the present disclosure.

The foregoing detailed description has been described with reference to various embodiments. However, those skilled in the art will recognize that various amendments and changes may be made without departing from the scope of the present disclosure. Therefore, considerations for the present disclosure are to be in an illustrative rather than a restrictive sense, and all such modifications are intended to be included within the scope hereof. Similarly, advantages of various embodiments, other advantages, and solutions to problems have been described above. However, the benefits, advantages, solutions to problems, and any elements that contribute to these, or solutions that make them clearer are not to be construed as critical, essential, or necessary. As used herein, the terms "include" and "comprise", and any other variants thereof are intended to be non-exclusive, such that a process, method, article, or device that includes a list of elements includes not only these elements but also other elements that are not explicitly listed or do not belong to the process, method, system, article, or device. Further, the term "coupling" and any other variants thereof used herein refer to a physical connection, an electrical connection, a magnetic connection, an optical connection, a communicative connection, a functional connection, and/or any other connection.

Those skilled in the art will recognize that numerous changes may be made to the details of the above embodiments without departing from the basic principles of the present disclosure. Therefore, the scope of the present disclosure should be determined by the claims only.

**Claims**

1. A method for image reconstruction, comprising:

   acquiring a plurality of raw images of a sample of interest in a same field of view; and
   performing image reconstruction based on the plurality of raw images for the field of view to obtain a reconstructed image of the field of view.

2. The method for image reconstruction according to claim 1, wherein the raw images are acquired in the field of view after the sample of interest is illuminated with a patterned illumination light.

3. The method for image reconstruction according to claim 2, wherein the patterned illumination light is obtained by passing an illumination light through a light modulator, wherein the light modulator comprises a body, the body comprises a plurality of first regions and a plurality of second regions randomly distributed on the body, the first region has a first light transmittance, and the second region has a second light transmittance, the first light transmittance being different from the second light transmittance.

4. The method for image reconstruction according to claim 3, wherein the sample of interest is scanned step by step in the field of view according to a preset rule, and one of the raw images for the field of view is obtained for each scan.

5. The method for image reconstruction according to claim 4, wherein the light modulator is controlled to move step by step relative to the illumination light, and the scan is completed once each time the light modulator moves.

6. The method for image reconstruction according to claim 5, wherein the patterned illumination light changes once with each movement.

7. The method for image reconstruction according to any one of claims 1 to 6, wherein performing image reconstruction based on the plurality of raw images for the field of view to obtain the reconstructed image of the field of view, comprises:

   iterating one or more of a ground truth pattern of the sample of interest in the field of view, the patterned illumination light, and a description function of an optical system to update estimate values thereof; and
   obtaining, after determining that iterative convergence is achieved, the reconstructed image through calculation based on the estimate values of the ground truth pattern of the sample of interest in the field of view, the patterned illumination light, and the description function of the optical system.

8. The method for image reconstruction according to claim 7, wherein iterating the one or more of the ground truth pattern of the sample of interest in the field of view, the patterned illumination light, and the description function to update the estimate values thereof, comprises: iterating, based on a difference function, the one or more of the ground truth pattern of the sample of interest in the field of view, the patterned illumination light, and the description function to update the estimate values thereof, wherein the difference function is used for describing a difference between the acquired raw image and an estimate value of the raw image, and the estimate value of the raw image is calculated based on an estimate value of the ground truth pattern of the sample of interest in the field of view, an estimate value of the patterned illumination light, and an estimate value of the description function.

9. The method for image reconstruction according to claim 8, wherein iterating, based on the difference function, the one or more of the ground truth pattern of the sample of interest in the field of view, the patterned illumination light, and the description function to update the estimate values thereof, comprises:
   iterating, based on the difference function, the one or more of the ground truth pattern of the sample of interest in the field of view, the patterned illumination light, and the description function by using a random gradient descent algorithm to update the estimate values thereof.

10. The method for image reconstruction according to claim 9, wherein iterating, based on the difference function, the one or more of the ground truth pattern of the sample of interest in the field of view, the patterned illumination light, and the description function by using the random gradient descent algorithm to update the estimate values thereof, comprises:

$$imgSR^{update} = imgSR - k_1 \cdot \frac{\partial C_i}{\partial imgSR}$$

and/or,

$$rpSR^{update} = rpSR - k_2 \cdot \frac{\partial C_i}{\partial rpSR}$$

and/or,

$$PSFSR^{update} = PSFSR - k_3 \cdot \frac{\partial C_i}{\partial PSFSR}$$

wherein *imgSR, rpSR,* and *PSFSR* represent the estimate value of the ground truth pattern of the sample of interest in the field of view, the estimate value of the patterned illumination light, and the estimate value of the description function, respectively; *imgSR^{update}, rpSR^{update}*, and *PSFSR^{update}* represent an updated estimate value of the ground truth pattern of the sample of interest in the field of view, an updated estimate value of the patterned illumination light, and an updated estimate value of the description function in one iteration process, respectively; $C_i$ represents a difference between an i-th raw image acquired in the field of view and an estimate value of the raw image; and $k_1$, $k_2$, and $k_3$ are iterative steps.

11. The method for image reconstruction according to claim 9, wherein iterating, based on the difference function, the one or more of the ground truth pattern of the sample of interest in the field of view, the patterned illumination light, and the description function by using the random gradient descent algorithm to update the estimate values thereof, comprises:

$$imgSR^{update} = imgSR - sf_1 \cdot k_1 \cdot \frac{\partial C_i}{\partial imgSR}$$

and/or,

$$rpSR^{update} = rpSR - sf_2 \cdot k_2 \cdot \frac{\partial C_i}{\partial rpSR}$$

and/or,

$$PSFSR^{update} = PSFSR - sf_3 \cdot k_3 \cdot \frac{\partial C_i}{\partial PSFSR}$$

wherein *imgSR, rpSR,* and *PSFSR* represent the estimate value of the ground truth pattern of the sample of interest in the field of view, the estimate value of the patterned illumination light, and the estimate value of the description function, respectively; *imgSR^{update}, rpSR^{update}*, and *PSFSR^{update}* represent an updated estimate value of the ground truth pattern of the sample of interest, an updated estimate value of the patterned illumination light, and an updated estimate value of the description function in one iteration process, respectively; $C_i$ represents a difference between an i-th raw image acquired in the field of view and an estimate value of the raw image; $k_1$, $k_2$, and $k_3$ are iterative steps; and $sf_1$, $sf_2$, and $sf_3$ are step coefficients of the iterative step $k_1$, the iterative step $k_2$, and the iterative step $k_3$, respectively.

12. The method for image reconstruction according to claim 10 or 11, wherein the iterative step $k_1$ is defined by an initial estimate value of the patterned illumination light; and/or, the iterative step $k_2$ is defined by an initial estimate value of the ground truth pattern of the sample of interest; and/or, the iterative step $k_3$ is defined by an initial estimate value of the raw image.

**13.** The method for image reconstruction according to claim 12, wherein

$$k_1 = \frac{1}{|\max(rpSR)|^2}$$

and/or,

$$k_2 = \frac{1}{|\max(imgSR)|^2}$$

and/or,

$$k_3 = \frac{1}{|max(\mathcal{F}^2(imgSR \cdot rpSR))|^2}$$

wherein $\mathcal{F}^2$ represents a two-dimensional Fourier transform.

**14.** The method for image reconstruction according to claim 11, wherein the step coefficient $sf_1$, the step coefficient $sf_2$, and the step coefficient $sf_3$ are selected from a preset step coefficient set.

**15.** The method for image reconstruction according to claim 8, wherein the difference function is $C_i = |imgDF_i - (imgSR \cdot rpSR) * PSFSR|^2$, wherein $C_i$ represents a difference between an i-th raw image acquired in the field of view and an estimate value of the raw image, and $imgDF_i$ represents the i-th raw image acquired in the field of view; and *imgSR, rpSR,* and *PSFSR* represent the estimate value of the ground truth pattern of the sample of interest in the field of view, the estimate value of the patterned illumination light, and the estimate value of the description function, respectively.

**16.** The method for image reconstruction according to claim 7, 8, 9, 10, 12, or 13, further comprising:

obtaining a cost function, wherein the cost function is used for describing a total amount of differences between the plurality of raw images acquired in the field of view and estimate values of the raw images, respectively; and
determining whether iterative convergence is achieved based on the cost function.

**17.** The method for image reconstruction according to claim 16, wherein the cost function is: $\sum_1^N C_i = \sum_1^N |imgDF_i - (imgSR \cdot rpSR) * PSFSR|^2$ , wherein $imgDF_i$ represents the i-th raw image acquired in the field of view, *imgSR, rpSR,* and *PSFSR* represent the estimate value of the ground truth pattern of the sample of interest in the field of view, the estimate value of the patterned illumination light, and the estimate value of the description function, respectively, and N represents a number of the raw images acquired in the field of view.

**18.** The method for image reconstruction according to claim 7, 8, 9, 11, 12, 13, or 14, further comprising:

obtaining a functional relationship between a minimum number of iterations required to achieve iterative convergence and a step coefficient, wherein the step coefficient comprises one or more of the step coefficient $sf_1$, the step coefficient $sf_2$, and the step coefficient $sf_3$;
selecting a value of the step coefficient by traversing the step coefficient set to determine the minimum number of iterations based on the functional relationship; and
determining, when a number of iterations reaches the minimum number of iterations, that iterative convergence is achieved.

**19.** The method for image reconstruction according to claim 18, wherein the functional relationship is established based on the step coefficient, the number of iterations, and an image evaluation index of an image after iteration.

**20.** The method for image reconstruction according to claim 7, 8, 9, 10, 12, or 13, further comprising, for each iteration:

obtaining an image after a current iteration through calculation;

calculating an image evaluation index based on the image after the current iteration;

calculating a convergence index after the current iteration based on the image evaluation index of the image after the current iteration; and

determining whether iterative convergence is achieved based on the convergence index after the current iteration.

21. The method for image reconstruction according to claim 20, wherein calculating the convergence index after the current iteration based on the image evaluation index of the image after the current iteration, comprises:

calculating a difference between the image evaluation index of the image after the current iteration and an image evaluation index of an image after a previous iteration, wherein the difference is referred to as a first difference;

calculating a difference between the image evaluation index of the image after the current iteration and an image evaluation index of an image after a first iteration, wherein the difference is referred to as a second difference; and

calculating a difference between the first difference and the second difference as the convergence index.

22. The method for image reconstruction according to claim 21, wherein calculating the difference between the first difference and the second difference, comprises: calculating a percentage of the first difference and the second difference.

23. The method for image reconstruction according to claim 22, wherein it is determined that iterative convergence is achieved when the percentage is greater than a preset percentage.

24. The method for image reconstruction according to claim 19 or 20, wherein the image evaluation index comprises a non-reference image evaluation index.

25. The method for image reconstruction according to claim 24, wherein the non-reference image evaluation index comprises a coefficient of variation and/or image entropy.

26. The method for image reconstruction according to any one of claims 7 to 14, wherein the initial estimate value of the ground truth pattern of the sample of interest in the field of view is: $imgSR = \frac{\sum_1^N imgDF_i}{N}$ , wherein $imgDF_i$ represents the i-th raw image acquired in the field of view, and N represents a number of the raw images acquired in the field of view; or, $imgSR = imgDF_1$, wherein $imgDF_1$ represents a first raw image acquired in the field of view, wherein $N \geq 1$;

and/or,

the initial estimate value of the patterned illumination light is:

$$rpSR = 1$$

and/or,

an initial estimate value of the description function is:

$$PSFSR = \mathcal{F}^2(imgSR)|_{f_{cutoff} \leq f_{q \cdot \max|\mathcal{F}^2(imgSR)|}}$$

which represents that the initial estimate value $imgSR$ of the ground truth pattern of the sample of interest is subjected to a two-dimensional Fourier transform to obtain a spectrum, and a cutoff frequency $f_{cutoff}$ of $PSFSR$ is at a spectrum position $f_{q \cdot \max|\mathcal{F}^2(imgSR)|}$ where an amplitude is $q$ times a maximum amplitude of the spectrum, wherein $\mathcal{F}^2$ represents the two-dimensional Fourier transform, and $q$ is a constant.

27. The method for image reconstruction according to claim 26, wherein $0 < q \leq 10\%$; and optionally, $q = 5\%$.

28. The method for image reconstruction according to claim 7, wherein the description function of the optical system is a point spread function corresponding to an optical transfer function of the optical system; and optionally, the optical transfer function of the optical system is generated based on a numerical aperture NA value of an objective lens of the optical system.

29. The method for image reconstruction according to any one of claims 3 to 28, wherein the light modulator comprises a body, the body comprises a first region and a second region arranged on the body, the first region has a first light transmittance, and the second region has a second light transmittance, the first light transmittance being different from the second light transmittance.

30. The method for image reconstruction according to claim 29, wherein the first light transmittance is greater than a first light transmittance threshold, and the second light transmittance is less than a second light transmittance threshold, the first light transmittance threshold being greater than or equal to the second light transmittance threshold.

31. The method for image reconstruction according to claim 29 or 30, wherein the first light transmittance is greater than the second light transmittance, and a difference therebetween is greater than a first threshold.

32. The method for image reconstruction according to any one of claims 29 to 31, wherein the first light transmittance is greater than 70%, 80%, 90%, or 95%; and/or the second light transmittance is less than 30%, 20%, 10%, or 5%.

33. The method for image reconstruction according to claim 32, wherein the first region is a light-passing region, and the first light transmittance is 100%; and/or the second region is a light-blocking region, and the second light transmittance is 0%.

34. The method for image reconstruction according to any one of claims 29 to 33, wherein the first region has a first refractive index, and the second region has a second refractive index; and the first refractive index is less than a first refractive index threshold, and the second refractive index is greater than a second refractive index threshold, the first refractive index threshold being less than or equal to the second refractive index threshold.

35. The method for image reconstruction according to claim 34, wherein the first refractive index is less than the second refractive index, and a difference therebetween is greater than a second threshold.

36. The method for image reconstruction according to any one of claims 29 to 35, wherein the first region has a first reflectance ratio, and the second region has a second reflectance ratio; and the first reflectance ratio is less than a first reflectance ratio threshold, and the second reflectance ratio is greater than a second reflectance ratio threshold, the first reflectance ratio threshold being less than or equal to the second reflectance ratio threshold.

37. The method for image reconstruction according to claim 36, wherein the first reflectance ratio is less than the second reflectance ratio, and a difference therebetween is greater than a third threshold.

38. The light modulator of any one of claims 29 to 37, wherein the first region has a first thickness, and the second region has a second thickness; and the first thickness is less than a first thickness threshold, and the second thickness is greater than a second thickness threshold, the first thickness threshold being less than or equal to the second thickness threshold.

39. The method for image reconstruction according to claim 38, wherein the first thickness is less than the second thickness, and a difference therebetween is greater than a fourth threshold.

40. The method for image reconstruction according to any one of claims 1 to 39, wherein there are a plurality of first regions and a plurality of second regions.

41. The method for image reconstruction according to claim 40, wherein the plurality of first regions and the plurality of second regions are irregularly distributed on the body.

42. The method for image reconstruction according to claim 41, wherein the plurality of first regions and the plurality of second regions are randomly distributed on the body.

43. The method for image reconstruction according to any one of claims 40 to 42, wherein a total area of the plurality of first regions and a total area of the plurality of second regions have a ratio ranging from 1:3 to 3:1.

44. The method for image reconstruction according to claim 43, wherein the total area of the plurality of first regions and the total area of the plurality of second regions have a ratio of 1:1.

45. The method for image reconstruction according to any one of claims 40 to 44, wherein the body is provided with a first quantity range of the first regions randomly distributed within each unit area region; and/or the body is provided with a second quantity range of the second regions randomly distributed within each unit area region.

46. The method for image reconstruction according to claim 45, wherein the first quantity range is 120 to 1500; and/or the second quantity range is 120 to 1500.

47. The method for image reconstruction according to any one of claims 40 to 46, wherein the plurality of first regions and the plurality of second regions are distributed on the body in a manner where an optical modulation degree is greater than a fifth threshold.

48. The method for image reconstruction according to claim 47, wherein the optical modulation degree is equal to a ratio of a difference in light intensity to a sum of light intensity for the body or a specified row of the body, wherein the difference in light intensity is a difference between a maximum light intensity and a minimum light intensity, and the sum of light intensity is a sum of the maximum light intensity and the minimum light intensity.

49. The method for image reconstruction according to claim 40, wherein shapes of the first region and the second region are identical.

50. The light modulator according to claim 49, wherein the first region and the second region are both square, or both triangular.

51. The method for image reconstruction according to any one of claims 1 to 50, wherein the body is sheet-like.

52. The method for image reconstruction according to any one of claims 1 to 51, wherein a first material is provided on the body to form the first region, and a second material is provided to form the second region.

53. The method for image reconstruction according to any one of claims 1 to 51, wherein the body is a transparent body, wherein a second material is provided on the transparent body to form the second region, and regions on the transparent body where the second material is not provided form the first region.

54. The method for image reconstruction according to claim 52 or 53, wherein the second material is metal Cr.

55. The method for image reconstruction according to any one of claims 1 to 54, wherein acquiring the plurality of raw images of the sample of interest in the same field of view, comprises:

projecting the illumination light onto the sample of interest to form one set of patterned illumination lights, wherein the one set of patterned illumination lights comprises a plurality of different patterned illumination lights, and the plurality of different patterned illumination lights correspond one-to-one with a plurality of raw images comprised in one set of images for the field of view.

56. The method for image reconstruction according to claim 55, wherein when acquiring one set of images for a same field of view, the illumination light is modulated by the light modulator and is projected onto the sample of interest, forming the one set of patterned illumination lights.

57. The method for image reconstruction according to claim 56, wherein when acquiring one set of images for a same field of view, the light modulator is controlled to move according to a preset movement rule in order to change a patterned illumination light projected onto the field of view, thereby resulting in the one set of patterned illumination lights for the field of view.

58. The method for image reconstruction according to claim 56, wherein when acquiring one set of images for a same field of view, the sample of interest is controlled to move according to a preset movement rule in order to change a patterned illumination light projected onto the field of view, thereby resulting in the one set of patterned illumination lights for the field of view.

59. The method according to claim 57 or 58, wherein the preset movement rule is determined by a preset pattern of the sample of interest on a surface of a chip.

60. The method for image reconstruction according to claim 59, wherein the preset movement rule comprises a trajectory composed of one or more step paths; and when the preset pattern is a quadrilateral-arranged dot matrix, the preset movement rule comprises: turning points of the trajectory as a whole forming a quadrilateral or a triangle; or the trajectory being parallel to one side of the quadrilateral.

61. The method for image reconstruction according to claim 60, wherein the turning points of the trajectory as a whole forming a quadrilateral or a triangle comprises any of the following:

nodes of the trajectory comprise a center of the quadrilateral and nodes located on each side of the quadrilateral, and starting from the center of the quadrilateral, the trajectory reaches a node on a side of the quadrilateral, and then, from the node, traverses each node on sides of the quadrilateral in one direction, or vice versa, wherein the one direction is a clockwise direction or
a counterclockwise direction;
a Z-shaped or zigzag-shaped trajectory;
a square-wave-shaped trajectory;
nodes of the trajectory comprise four vertices of the quadrilateral, and starting from one vertex of the quadrilateral, the trajectory reaches an opposite vertex, then to an adjacent vertex of the opposite vertex, and then to an opposite vertex of the adjacent vertex;
nodes of the trajectory comprise a center and four vertices of the quadrilateral, and starting from one vertex of the quadrilateral, the trajectory passes through the center to an opposite vertex, then to an adjacent vertex of the opposite vertex, and then to an opposite vertex of the adjacent vertex;
nodes of the trajectory comprise a center and four vertices of the quadrilateral, and starting from the center of the quadrilateral, the trajectory reaches one vertex of the quadrilateral, and then traverses each vertex on sides of the quadrilateral in one direction, wherein the one direction is a clockwise direction or a counterclockwise direction;
nodes of the trajectory comprise four vertices of the quadrilateral, and starting from one vertex of the quadrilateral, the trajectory traverses each vertex on sides of the quadrilateral in one direction, wherein the one direction is a clockwise direction or a counterclockwise direction;
nodes of the trajectory comprise three vertices of the triangle, and starting from one vertex of the triangle, the trajectory traverses each vertex of the triangle in one direction, wherein the one direction is a clockwise direction or a counterclockwise direction, and
nodes of the trajectory comprise three vertices of the triangle and one or more nodes located on one or more sides of the triangle, and starting from one node of the triangle, the trajectory traverses nodes on the triangle in one direction, wherein the one direction is a clockwise direction or a counterclockwise direction.

62. The method for image reconstruction according to claim 59, wherein the preset movement rule comprises a trajectory composed of one or more step paths; and when the preset pattern is a triangle-arranged dot matrix, the preset movement rule comprises: turning points of the trajectory as a whole forming a hexagon or triangle, or the trajectory being parallel to one side of the triangle.

63. The method for image reconstruction according to claim 62, wherein the turning points of the trajectory as a whole forming a hexagon or triangle, comprises the following:

nodes of the trajectory comprise a center and six vertices of the hexagon, and starting from the center of the hexagon, the trajectory reaches one of the vertices, and then traverses each vertex of the hexagon in one direction, or vice versa, wherein the one direction is a clockwise direction or a counterclockwise direction;
nodes of the trajectory comprise all vertices of the hexagon, and starting from one vertex of the hexagon, the trajectory traverses each vertex of the hexagon in one direction, wherein the one direction is a clockwise direction or a counterclockwise direction;
nodes of the trajectory comprise three vertices of the triangle, and starting from one vertex of the triangle, the trajectory traverses each vertex of the triangle in one direction, wherein the one direction is a clockwise direction or a counterclockwise direction, and
nodes of the trajectory comprise a center and three vertices of the triangle, and starting from the center of the triangle, the trajectory reaches one of the vertices, and then traverses each vertex of the triangle in one direction, or vice versa, wherein the one direction is a clockwise direction or a counterclockwise direction.

64. The method for image reconstruction according to any one of claims 57 to 63, wherein the preset movement rule comprises a first point, a second point, and a trajectory between the first point and the second point; and under each field of view, the preset movement rule is: starting from the first point and moving to the second point along a preset trajectory between the first point and the second point.

65. The method for image reconstruction according to claim 64, wherein for any two adjacent fields of view: while acquiring one set of images for one field of view, the light modulator is controlled to move step by step from the first point to the second point according to the preset movement rule to acquire the one set of images for the field of view; after a last raw image for the field of view is acquired, the light modulator is controlled to move back to the first point, and the sample of interest is controlled to move to switch to a next field of view; and while acquiring one set of images for the next field of view, the light modulator is controlled to move step by step from the first point to the second point according to the preset movement rule to acquire the one set of images for the next field of view.

66. The method for image reconstruction according to claim 64, wherein for any two adjacent fields of view: while acquiring one set of images for one field of view, the sample of interest is controlled to move step by step from the first point to the second point according to the preset movement rule to acquire the one set of images for the field of view; after a last raw image for the field of view is acquired, the sample of interest is controlled to move back to the first point, and the sample of interest is controlled to move to switch to a next field of view; and while acquiring one set of images for the next field of view, the sample of interest is controlled to move step by step from the first point to the second point according to the preset movement rule to acquire the one set of images for the next field of view.

67. The method for image reconstruction according to any one of claims 57 to 63, wherein the preset movement rule comprises a first point, a second point, and a trajectory between the first point and the second point; and in a case of two adjacent fields of view, the preset movement rule for one field of view is: moving from the first point to the second point along a preset trajectory between the first point and the second point; and the preset movement rule for the other field of view is: moving from the second point to the first point along the preset trajectory between the first point and the second point.

68. The method for image reconstruction according to claim 67, wherein for any two adjacent fields of view:

while acquiring one set of images for one field of view, if the preset movement rule for the field of view is to move from the first point to the second point along the preset trajectory between the first point and the second point, then the light modulator is controlled to move step by step from the first point to the second point according to the preset movement rule to acquire the one set of images for the field of view; after a last raw image for the field of view is acquired, the sample of interest is controlled to move to switch to a next field of view; while acquiring one set of images for the next field of view, the light modulator is controlled to move step by step from the second point to the first point according to the preset movement rule to acquire the one set of images for the next field of view; and while acquiring one set of images for one field of view, if the preset movement rule for the field of view is to move from the first point to the second point along the preset trajectory between the first point and the second point, then the light modulator is controlled to move step by step from the second point to the first point according to the preset movement rule to acquire the one set of images for the field of view; after a last raw image for the field of view is acquired, the sample of interest is controlled to move to switch to a next field of view; and while acquiring one set of images for the next field of view, the light modulator is controlled to move step by step from the first point to the second point according to the preset movement rule to acquire the one set of images for the next field of view.

69. The method for image reconstruction according to claim 67, wherein for any two adjacent fields of view:

while acquiring one set of images for one field of view, if the preset movement rule for the field of view is to move from the first point to the second point along the trajectory between the first point and the second point, then the sample of interest is controlled to move step by step from the first point to the second point according to the preset movement rule to acquire the one set of images for the field of view; after a last raw image for the field of view is acquired, the sample of interest is controlled to move to switch to a next field of view; and while acquiring one set of images for the next field of view, the sample of interest is controlled to move step by step from the second point to the first point according to the preset movement rule to acquire the one set of images for the next field of view; and while acquiring one set of images for one field of view, if the preset movement rule for the field of view is to move from the second point to the first point along the trajectory between the first point and the second point, then the sample of interest is controlled to move step by step from the second point to the first point according to the preset movement rule to acquire the one set of images for the field of view; after a last raw image for the field of view is acquired, the sample of interest is controlled to move to switch to a next field of view; and while acquiring one set of

# EP 4 648 002 A1

images for the next field of view, the sample of interest is controlled to move step by step from the first point to the second point according to the preset movement rule to acquire the one set of images for the next field of view.

**70.** The method for image reconstruction according to claim 56, wherein the light modulator comprises a body, a plurality of first regions, and a plurality of second regions, the first region has a first light transmittance, and the second region has a second light transmittance, wherein the first light transmittance is different from the second light transmittance, and the plurality of first regions and the plurality of second regions are randomly distributed on the body; and the patterned illumination light projected by the light modulator is a random patterned illumination light.

**71.** The method for image reconstruction according to any one of claims 56, or 58 to 70, wherein for two adjacent raw images in the one set of images of any of the fields of view, the following actions are performed:

action one: controlling a camera to expose and acquire one raw image for the field of view under a current patterned illumination light;
action two: processing the acquired one raw image using a reconstruction algorithm; and
action three: controlling the light modulator to move to a next node under the preset movement rule to update the patterned illumination light once, so as to prepare for acquisition of a next raw image for the same field of view, wherein
the action two and the action three overlap at least partially in timing and both occur after the action one.

**72.** The method for image reconstruction according to claim 71, further comprising:

action four: controlling the camera to expose and acquire the next raw image for the field of view;
action five: processing the acquired next raw image using a reconstruction algorithm; and
in a case where the next raw image is not a last raw image for the field of view, performing action six: controlling the light modulator to move to a next node under the preset movement rule to update the patterned illumination light once, wherein
the action five and the action six overlap at least partially in timing and both occur after the action four.

**73.** The method for image reconstruction according to claim 72, wherein after performing the action five, if the next raw image is the last raw image for the field of view, action seven is performed: controlling the sample of interest to move to switch to a next field of view, so as to perform parallel image acquisition and reconstruction algorithm for the next field of view until a current field of view is a last field of view, wherein
the action seven and the action five overlap at least partially in timing and both occur after the action four.

**74.** The method for image reconstruction according to claim 73, wherein when, under each field of view, the preset movement rule is: moving from the first point to the second point along the trajectory between the first point and the second point, then the action seven further comprises:

controlling the light modulator to move from the second point back to the first point;
or alternatively,
when, in a case of two adjacent fields of view, the preset movement rule for one field of view is:

moving from the first point to the second point along the trajectory between the first point and the second point, and the preset movement rule for the other field of view is: moving from the second point to the first point along the trajectory between the first point and the second point,
then the action seven further comprises: controlling the light modulator to remain stationary at a current position.

**75.** The method for image reconstruction according to any one of claims 57 to 70, wherein for two adjacent raw images in the one set of images of any of the fields of view:

action one: controlling a camera to expose and acquire one raw image for the field of view under a current patterned illumination light;
action two: processing the acquired one raw image using a reconstruction algorithm; and
action three: controlling the sample of interest to move to a next node under the preset movement rule to update the patterned illumination light once, so as to prepare for acquisition of a next raw image for the same field of view, wherein

the action two and the action three overlap at least partially in timing and both occur after the action one.

76. The method for image reconstruction according to claim 75, further comprising:

action four: controlling the camera to expose and acquire the next raw image for the field of view;
action five: processing the acquired next raw image using a reconstruction algorithm; and
in a case where the next raw image is not a last raw image for the field of view, performing action six: controlling the sample of interest to move to a next node under the preset movement rule to update the randomly patterned illumination light once, wherein
the action five and the action six overlap at least partially in timing and both occur after the action four.

77. The method for image reconstruction according to claim 76, wherein after performing the action four, if the next raw image is the last raw image for the field of view, action seven is performed: controlling the sample of interest to move to switch to a next field of view, so as to perform parallel image acquisition and reconstruction algorithm for the next field of view until a current field of view is a last field of view, wherein
the action seven and the action five overlap at least partially in timing and both occur after the action four.

78. The method for image reconstruction according to claim 77, wherein when, under each field of view, the preset movement rule is: moving from the first point to the second point along the trajectory between the first point and the second point, then the action seven further comprises:

after controlling the sample of interest to move to switch to the next field of view, controlling the sample of interest to move from the second point back to the first point;
or alternatively,
when, in a case of two adjacent fields of view, the preset movement rule for one field of view is: moving from the first point to the second point along the trajectory between the first point and the second point, and the preset movement rule for the other field of view is: moving from the second point to the first point along the trajectory between the first point and the second point, then the action seven further comprises: after controlling the sample of interest to move to switch to the next field of view, controlling the sample of interest to remain stationary at a current position.

79. The method for image reconstruction according to any one of claims 1 to 78, further comprising:

repeatedly updating an illumination light projected onto a field of view of a sample of interest by using a microscopic imaging system, and performing multiple scanning imaging operations on the field of view to obtain a plurality of raw images, wherein each time the illumination light is updated, one scanning is performed, and one scanning results in one raw image; and
performing image reconstruction based on the raw image to obtain a reconstructed image with higher resolution, wherein the image reconstruction is performed in parallel with the multiple scanning imaging operations and begins based on a portion of the raw image.

80. The method for image reconstruction according to claim 79, wherein
performing image reconstruction based on the raw image to obtain the reconstructed image with higher resolution, comprises:
each time a raw image for the field of view is acquired, processing the raw image using a reconstruction algorithm, and when a last raw image for the field of view is acquired, processing the last raw image using the reconstruction algorithm to obtain the reconstructed image for the field of view.

81. The method for image reconstruction according to claim 79 or 80, wherein the reconstruction algorithm comprises an iterative algorithm; and for a same field of view,
a first raw image for the field of view acquired by scanning is used as an initial estimate value of the reconstructed image to perform the iterative algorithm.

82. The method for image reconstruction according to claim 80 or 81, further comprising:
obtaining, after processing the last raw image for the field of view using a reconstruction algorithm to obtain the reconstructed image for the field of view, a detection result of the sample of interest based on the reconstructed image.

83. The method for image reconstruction according to any one of claims 79 to 82, wherein for any of the fields of view: a patterned illumination light that illuminates the sample of interest in the field of view is updated once, and image acquisition is controlled to obtain one raw image from a current scan of the field of view.

84. The method for image reconstruction according to claim 83, wherein the patterned illumination light is obtained by passing an illumination light through a light modulator, and the light modulator is controlled to move relative to the illumination light, such that each movement can update the patterned illumination light once.

85. The method for image reconstruction according to claim 84, wherein for any of the fields of view: multiple scans of the field of view are completed by controlling the light modulator to move relative to the illumination light according to a preset rule.

86. The method for image reconstruction according to any one of claims 83 to 85, wherein for two adjacent raw images of any of the fields of view, the following actions are performed:

action one: controlling a camera to expose and acquire one raw image for the field of view under a current patterned illumination light;
action two: processing the acquired one raw image using a reconstruction algorithm; and
action three: controlling the light modulator to move once to update the patterned illumination light once, so as to prepare for acquisition of a next raw image for the same field of view,
wherein
the action two and the action three overlap at least partially in timing and both occur after the action one.

87. The method for image reconstruction according to claim 86, further comprising:

action four: controlling the camera to expose and acquire the next raw image for the field of view;
action five: processing the acquired next raw image using a reconstruction algorithm; and
in a case where the next raw image is not a last raw image for the field of view, performing action six: controlling the light modulator to move once more to update the patterned illumination light once, wherein
the action five and the action six overlap at least partially in timing and both occur after the action four.

88. The method for image reconstruction according to claim 87, wherein after performing the action four, if the next raw image is the last raw image for the field of view, action seven is performed: controlling the sample of interest to move to switch to a next field of view, so as to perform parallel image acquisition and reconstruction algorithm for the next field of view until a current field of view is a last field of view, wherein
the action seven and the action five overlap at least partially in timing and both occur after the action four.

89. The method for image reconstruction according to claim 88, wherein the preset movement rule comprises a first point, a second point, and a trajectory between the first point and the second point; and

under each field of view, the preset movement rule is: moving from the first point to the second point along the trajectory between the first point and the second point; and the action seven further comprises: controlling the light modulator to move from the second point back to the first point;
or alternatively,
in a case of two adjacent fields of view, the preset movement rule for one field of view is: moving from the first point to the second point along the trajectory between the first point and the second point, and the preset movement rule for the other field of view is: moving from the second point to the first point along the trajectory between the first point and the second point; and the action seven further comprises: controlling the light modulator to remain stationary at a current position.

90. The method for image reconstruction according to any one of claims 83 to 85, wherein for two adjacent raw images of any of the fields of view:

during a first period, control is executed to perform image acquisition to obtain one raw image for the field of view under a current patterned illumination light;
during a second period, the patterned illumination light is updated once to prepare for acquisition of a next raw image for the same field of view; and
during a third period, the one raw image acquired during the first period is processed using a reconstruction algorithm, wherein

the second period and the third period at least partially overlap, and both occur after the first period.

91. The method for image reconstruction according to claim 90, wherein during a fourth period, control is executed to perform image acquisition to obtain the next raw image;

during a fifth period, in a case where the next raw image is not a last raw image for the field of view, then the patterned illumination light is updated once to prepare for acquisition of a subsequent raw image for the same field of view; and
during a sixth period, the next raw image acquired during the fourth period is input for processing using a reconstruction algorithm, wherein
the fourth period follows the third period; and the fifth period and the sixth period at least partially overlap, and both occur after the fourth period.

92. The method for image reconstruction according to claim 91, wherein during the fifth period, if the next raw image is the last raw image for the field of view, then a next field of view is switched to, so as to perform parallel image acquisition and reconstruction algorithm for the next field of view, until a current field of view is a last field of view.

93. The method for image reconstruction according to claim 79, wherein upon acquiring a last raw image for a field of view, a next field of view is switched to, so as to perform parallel image acquisition and reconstruction algorithm for the next field of view, until a current field of view is a last field of view.

94. The method for image reconstruction according to claim 88, 92, or 93, wherein a field of view is switched to a next field of view by moving a stage carrying the sample of interest.

95. The method for image reconstruction according to any one of claims 1 to 94, wherein acquiring the plurality of raw images of the sample of interest in the same field of view; and performing image reconstruction based on the plurality of raw images for the field of view to obtain the reconstructed image of the field of view, comprises:

performing a Fourier transform on the raw image to obtain a spectrum diagram; and
obtaining a characteristic frequency dimension value $f_{pattern}$ and/or a cutoff frequency dimension value $f_{cutoff}$ based on the spectrum diagram, wherein the characteristic frequency dimension value $f_{pattern}$ is a distance between a pixel of a characteristic frequency peak on the spectrum diagram and a center pixel of the spectrum diagram, and the cutoff frequency dimension value $f_{cutoff}$ is a distance between a pixel of a cutoff frequency on the spectrum diagram and the center pixel of the spectrum diagram; and determining dimensions of the reconstructed image based on the characteristic frequency dimension value $f_{pattern}$ and/or the cutoff frequency dimension value $f_{cutoff}$.

96. The method for image reconstruction according to claim 95, wherein
determining the dimensions of the reconstructed image based on the characteristic frequency dimension value $f_{pattern}$ and/or the cutoff frequency dimension value $f_{cutoff}$, comprises:

determining one or more frequency dimension ranges based on the characteristic frequency dimension value $f_{pattern}$ and/or the cutoff frequency dimension value $f_{cutoff}$, wherein the one or more frequency dimension ranges comprise one or more of 2 * $f_{pattern}$, 2 * $f_{cutoff}$, and ($f_{pattern}$ + $f_{cutoff}$); and
determining the dimensions of the reconstructed image based on the one or more frequency dimension ranges.

97. The method for image reconstruction according to claim 96, wherein determining the dimensions of the reconstructed image based on the one or more frequency dimension ranges, comprises:

the reconstructed image is quadrilateral, a length of one side of the reconstructed image is 2 * $f_{pattern}$ pixels, 2 * $f_{cutoff}$ pixels, or ($f_{pattern}$ + $f_{cutoff}$) pixels, and a length of an adjacent side of the reconstructed image is 2 * $f_{pattern}$ pixels, 2 * $f_{cutoff}$ pixels, or ($f_{pattern}$ + $f_{cutoff}$) pixels;
or, the reconstructed image is circular, and a diameter of the reconstructed image is 2 * $f_{pattern}$ pixels, 2 * $f_{cutoff}$ pixels, or ($f_{pattern}$ + $f_{cutoff}$) pixels.

98. The method for image reconstruction according to any one of claims 95 to 97, wherein determining the dimensions of the reconstructed image based on the characteristic frequency dimension value $f_{pattern}$ and/or the cutoff frequency dimension value $f_{cutoff}$, comprises:

determining the dimensions of the reconstructed image based on a constraint of a Fourier transform of a processor for reconstructing the raw image, and the characteristic frequency dimension value $f_{pattern}$ and/or the cutoff frequency dimension value $f_{cutoff}$.

99. The method for image reconstruction according to claim 98, wherein determining the dimensions of the reconstructed image based on the constraint of the Fourier transform of the processor for reconstructing the raw image, and the characteristic frequency dimension value $f_{pattern}$ and/or the cutoff frequency dimension value $f_{cutoff}$, comprises: determining the dimensions of the reconstructed image based on a minimum value greater than a first value among values satisfying the constraint of the Fourier transform, wherein the first value is 2 * $f_{pattern}$ pixels, 2 * $f_{cutoff}$ pixels, or ($f_{pattern}$ + $f_{cutoff}$) pixels.

100.
The method for image reconstruction according to claim 98, wherein
determining the dimensions of the reconstructed image based on the constraint of the Fourier transform of the processor for reconstructing the raw image, and the characteristic frequency dimension value $f_{pattern}$ and/or the cutoff frequency dimension value $f_{cutoff}$, comprises:
determining the dimensions of the reconstructed image based on a value closest to a first value among values satisfying the constraint of the Fourier transform, wherein the first value is 2 * $f_{pattern}$ pixels, 2 * $f_{cutoff}$ pixels, or ($f_{pattern}$ + $f_{cutoff}$) pixels.

101.
The method for image reconstruction according to claim 99 or 100, wherein the values satisfying the constraint of the Fourier transform are $2^a * 3^b$, wherein $a$ and $b$ are positive integers.

102.
The method for image reconstruction according to claim 97, wherein the reconstructed image is rectangular.

103.
The method for image reconstruction according to claim 98, wherein the processor is a graphic processing unit (GPU).

104.
An apparatus, comprising a memory and a processor, wherein the memory is configured to store programs, and the processor is configured to implement the method for image reconstruction according to any one of claims 1 to 103 by running the programs in the memory.

FIG. 1

FIG. 2(a)

FIG. 2(b)

FIG. 3

FIG. 4

FIG. 5

100

| Illumination apparatus 20 | Image acquisition apparatus 30 Camera 31 |
| --- | --- |
| Processor 40 | |

FIG. 6(a)

100

| Memory 41 |
| --- |
| Processor 40 |

FIG. 6(b)

Focusing
Illumination
Imaging

31a  36c  36b  34

Automatic focusing

33  50

39a  38a  37a  36a  20

21a

39b  38b  37b  32  10  23  21c  21b

39c  38c  37c  24

Sample

31d  31c  31b

FIG. 7

100

| Illumination apparatus 20 | Image acquisition apparatus 30 Camera 31 | Driving apparatus 60 |
| --- | --- | --- |
| Processor 40 | | |

FIG. 8

Acquiring images of a sample of interest under at least one field of view, where one set of images is acquired for each field of view, and one set of images includes a plurality of raw images ~ 1100

Projecting, when acquiring one set of images for the same field of view, the illumination light onto the sample of interest, thus forming one set of patterned illumination lights ~ 1200

FIG. 9

FIG. 10

FIG. 11(a)

| N | One-dimensional orthogonal scanning |
|---|---|
| 3 | $S_1=2, S_2=3$    $S_1=2, S_2=3$ |
| 2 | $S_1=1, S_2=2$    $S_1=1, S_2=2$ |

FIG. 11(b)

FIG. 12

| N | Two-dimensional triangular scanning |
|---|---|
| 7 | <br>$S_1=6, S_2=7$ |
| 6 | <br>$S_1=5, S_2=6$ |
| 4 | <br>$S_1=3, S_2=4$ |
| 3 | <br>$S_1=2, S_2=3$     $S_1=2, S_2=3$ |

FIG. 13(a)

| N | One-dimensional triangular scanning |
|---|---|
| 3 | <br>$S_1=2, S_2=3$    $S_1=2, S_2=3$    $S_1=2, S_2=3$ |
| 2 | <br>$S_1=1, S_2=2$    $S_1=1, S_2=2$    $S_1=1, S_2=2$ |

FIG. 13(b)

FIG. 14

FIG. 15(a)

FIG. 15(b)

Repeatedly updating the illumination light projected onto a field of view of a sample of interest by using a microscopic imaging system, and performing multiple scanning imaging operations on the field of view to obtain a plurality of raw images, where each time the illumination light is updated, one scanning is performed, and one scanning results in one raw image ~ 1300

Performing image reconstruction based on the raw image to obtain a reconstructed image with higher resolution, where the image reconstruction is performed in parallel with the multiple scanning imaging operations and begins based on a portion of the raw image ~ 1400

FIG. 16

Repeatedly updating the illumination light projected onto a field of view of a sample of interest by using a microscopic imaging system, and performing multiple scanning imaging operations on the field of view to obtain a plurality of raw images, where each time the illumination light is updated, one scanning is performed, and one scanning results in one raw image ~ 1300

Performing image reconstruction based on the raw image to obtain a reconstructed image with higher resolution, where the image reconstruction is performed in parallel with the multiple scanning imaging operations and begins based on a portion of the raw image ~ 1400

Switching, upon acquiring the last raw image for the field of view, to the next field of view to perform parallel image acquisition and reconstruction algorithms on the next field of view, until the current field of view is the last field of view ~ 1500

FIG. 17

Repeatedly updating the illumination light projected onto a field of view of a sample of interest by using a microscopic imaging system, and performing multiple scanning imaging operations on the field of view to obtain a plurality of raw images, where each time the illumination light is updated, one scanning is performed, and one scanning results in one raw image ~ 1300

Performing image reconstruction based on the raw image to obtain a reconstructed image with higher resolution, where the image reconstruction is performed in parallel with the multiple scanning imaging operations and begins based on a portion of the raw image ~ 1400

Obtaining, after inputting the last raw image of the same field of view for processing using a reconstruction algorithm to obtain a reconstructed image of the field of view, a detection result of the sample of interest based on the reconstructed image ~ 1600

FIG. 18

FIG. 19

| Acquiring a plurality of raw images of a sample of interest in the same field of view | ~ 1000 |

↓

| Performing image reconstruction based on the plurality of raw images for the field of view to obtain a reconstructed image of the field of view | ~ 2000 |

FIG. 20

| Iterating on one or more of the ground truth image of the sample of interest in the field of view, the patterned illumination light, and the description function of the optical system to update the estimate values thereof | ~ 2100 |

↓

| Calculating and obtaining, after determining that the iterative convergence has been achieved, the reconstructed image based on the estimate values of the ground truth image of the sample of interest in the field of view, the patterned illumination light, and the description function of the optical system | ~ 2200 |

FIG. 21

| Obtaining a cost function, where the cost function is used for describing the total amount of differences between the plurality of raw images acquired in the field of view and the estimate values of the raw images, respectively | ~ 2210 |

↓

| Determining whether iterative convergence has been achieved based on the cost function | ~ 2212 |

FIG. 22

| Obtaining the image after the current iteration through calculation | ~ 2220 |

↓

| Calculating an image evaluation index based on the image obtained after the current iteration | ~ 2222 |

↓

| Calculating a convergence index after the current iteration based on the image evaluation index of the image obtained after the current iteration | ~ 2224 |

↓

| Determining whether iterative convergence has been achieved based on the convergence index after the current iteration | ~ 2226 |

FIG. 23

FIG. 24

FIG. 25

FIG. 26

FIG. 27

FIG. 28

FIG. 29

FIG. 30

FIG. 31

FIG. 32

FIG. 33

| Spatial domain diagram | Enlarged diagram | Spectrum diagram |
|---|---|---|

FIG. 34

FIG. 35

## INTERNATIONAL SEARCH REPORT

| International application No. |
|---|
| **PCT/CN2024/070990** |

**A. CLASSIFICATION OF SUBJECT MATTER**

G06T3/4053(2024.01)i

According to International Patent Classification (IPC) or to both national classification and IPC

**B. FIELDS SEARCHED**

Minimum documentation searched (classification system followed by classification symbols)

IPC: G06T

Documentation searched other than minimum documentation to the extent that such documents are included in the fields searched

Electronic data base consulted during the international search (name of data base and, where practicable, search terms used)

CNABS; CNTXT; WPABS; DWPI; USTXT; WOTXT; EPTXT; CNKI; IEEE; Web of Science; 百度, BAIDU; 万方, WANFANG: 图像, 重建, 照明光, 光调控器, 透光率, 图案化, 移动, 扫描, 随机梯度下降, 估计, 频谱, image, reconstruction, light, modulator, pattern, moving, scanning, stochastic gradient descent, estimation, spectrum

**C. DOCUMENTS CONSIDERED TO BE RELEVANT**

| Category* | Citation of document, with indication, where appropriate, of the relevant passages | Relevant to claim No. |
|---|---|---|
| X | WO 2022120853 A1 (MGI TECHNOLOGY CO., LTD.) 16 June 2022 (2022-06-16) description, pages 2-13 | 1-104 |
| A | CN 111556257 A (BEIJING INSTITUTE OF TECHNOLOGY) 18 August 2020 (2020-08-18) entire document | 1-104 |
| A | CN 114626981 A (MGI TECHNOLOGY CO., LTD.) 14 June 2022 (2022-06-14) entire document | 1-104 |
| A | CN 114092325 A (SEIZET TECHNOLOGY (SHENZHEN) CO., LTD.) 25 February 2022 (2022-02-25) entire document | 1-104 |

☐ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **26 March 2024** | **08 April 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2024/070990**

| Patent document cited in search report | | | Publication date (day/month/year) | Patent family member(s) | | | Publication date (day/month/year) |
|---|---|---|---|---|---|---|---|
| WO | 2022120853 | A1 | 16 June 2022 | CN | 116490812 | A | 25 July 2023 |
| CN | 111556257 | A | 18 August 2020 | CN | 111556257 | B | 04 June 2021 |
| CN | 114626981 | A | 14 June 2022 | None | | | |
| CN | 114092325 | A | 25 February 2022 | CN | 114092325 | B | 16 August 2022 |

Form PCT/ISA/210 (patent family annex) (July 2022)